# EUROPÄISCHE PATENTSCHRIFT

(11) **EP 2 293 805 B1**
(45) Veröffentlichungstag und Bekanntmachung des Hinweises auf die Patenterteilung: **29.07.2015**
(21) Anmeldenummer: 09776551.5
(22) Anmeldetag: 22.04.2009
(51) Int. Cl.: A61K 36/185, A61K 36/68, A61P 11/00, A61K 36/73, A61K 36/49, A61K 36/09, A61K 36/48, A61K 36/61, A61K 36/45, A61K 36/328, A61K 36/537, A61K 36/736, A61K 36/53, A61K 36/739

(54) **LUTSCHZUSAMMENSETZUNG ZUR BEHANDLUNG VON ENTZÜNDLICHEN ERKRANKUNGEN DES MUND- UND RACHENRAUMS**
LOZENGE COMPOSITION FOR TREATING INFLAMMATORY DISEASES OF THE MOUTH AND PHARYNX
COMPOSITION À SUCER POUR LE TRAITEMENT D'AFFECTIONS INFLAMMATOIRES DE LA CAVITÉ BUCCALE ET DU PHARYNX

(30) Priorität: 30.06.2008 DE 202008008532 U
(43) Veröffentlichungstag der Anmeldung: 16.03.2011
(73) Patentinhaber: Maria Clementine Martin Klosterfrau Vertriebsgesellschaft mbH, 50670 Köln (DE)
(72) Erfinder: PLOCH, Michael, 16515 Oranienburg (DE)
(74) Vertreter: Von Rohr Patentanwälte Partnerschaft mbB
(86) Internationale Anmeldenummer: PCT/EP2009/002913
(87) Internationale Veröffentlichungsnummer: WO 2010/003472

(56) Entgegenhaltungen:
- EP-A- 0 870 507
- EP-A- 1 829 548
- GB-A- 2 093 696

## Beschreibung

Die vorliegende Erfindung betrifft den Bereich der entzündlichen Erkrankungen des Mund- und Rachenraums, beispielsweise als Begleiterscheinung von Erkältungen, sowie Hustenerkrankungen und Katharren der oberen Luftwege.

Insbesondere betrifft die vorliegende Erfindung eine insbesondere pharmazeutische Zusammensetzung in einer zum Lutschen geeigneten Darreichungsform, insbesondere auf Lutschtablettenbasis, wobei die Zusammensetzung eine Kombination mindestens einer ersten Wirkstoffkomponente, welche mindestens eine Gerbstoffdroge bzw. deren Extrakt enthält, mit mindestens einer zweiten Wirkstoffkomponente, welche mindestens eine Schleimdroge bzw. deren Extrakt enthält, umfaßt.

Des weiteren betrifft die vorliegende Erfindung eine Verpackungseinheit, welche die erfindungsgemäße Zusammensetzung in einer zur Einzeldosierung geeigneten Form, vorzugsweise in Form von Lutschtabletten, enthält.

Des weiteren betrifft die vorliegende Erfindung die Verwendung der erfindungsgemäßen Zusammensetzung zur topischen Behandlung von entzündlichen Erkrankungen des Mund- und Rachenraums, Husten und Katharren der oberen Luftwege bzw. zur Herstellung eines Medikamentes zur topischen Behandlung von entzündlichen Erkrankungen des Mund- und Rachenraums, Husten und Katharren der oberen Luftwege.

Entzündliche Erkrankungen des Mund- und Rachenraums, d. h. Entzündungen im Bereich der Mundhöhle und des Hals-/Rachenraums, treten oftmals als Begleiterscheinung von Erkältungserkrankungen und grippalen Infekten, aber auch als eigenständige Erkrankungen auf. Solche entzündlichen Prozesse des Mund- und Rachenraums sind oftmals mit einer für den betroffenen Patienten unangenehmen Schmerzsymptomatik verbunden. Nichtbeschränkende Beispiele für derartige entzündliche Erkrankungen des Hals-/Rachenraums sind Halsentzündungen, insbesondere Angina, Entzündungen des Kehlkopfs (Laryngitis), Entzündungen der Rachenschleimhaut (Pharyngitis) und Entzündungen der Rachenmandeln (Tonsillitis). Solche Erkrankungen des Hals-/Rachenraums sind oftmals von Schluckbeschwerden begleitet. Auch Entzündungen im Bereich der Mundhöhle, wie z. B. Stomatitis, Gingivitis, Mundschleimhautläsionen und dergleichen, weisen eine für den Betroffenen unangenehme Symptomatik auf. Für weitere diesbezügliche Einzelheiten zu den vorgenannten Erkrankungen kann beispielsweise auf Roche-Lexikon Medizin, 3. Auflage, 1993, Urban & Schwarzenberg, München/Wien/Baltimore, sowie auf Pschyrembel, Medizinisches Wörterbuch, 257. Auflage, 1993, Nikol Verlagsgesellschaft mbH, Hamburg, verwiesen werden. Der Begriff "entzündliche Erkrankungen des Mund- und Rachenraums" ist somit sehr weit zu verstehen und umfaßt sämtliche entzündlichen Erkrankungen, welche im Bereich der Mundhöhle, des Rachens und des Halsraums auftreten können.

Die Behandlung entzündlicher Erkrankungen des Mund- und Rachenraums erfolgt - in Abhängigkeit von der Schwere des Krankheitsbildes - im allgemeinen durch topische und gegebenenfalls zusätzliche systemische Therapie in schwerwiegenderen Fällen. Während in schwereren Fällen systemisch Antibiotika verabreicht werden, kommen unterstützend und in leichteren Fällen unter Umständen alleinig zur topischen, symptomatischen Behandlung oftmals Antiseptika und entzündungshemmende Stoffe (so z. B. Antiphlogistika, antiinflammatorisch wirkende Mittel, Lokalantibiotika etc.) zum Einsatz, welche beispielsweise in Form von Rachenspülungen, Sprays oder Lutschtabletten verabreicht werden können.

Als Wirkstoff zur topischen Behandlung von entzündlichen Prozessen des Mund- und Rachenraums hat sich insbesondere 1-Hexadecylpyridiniumchlorid (internationaler Freiname: "Cetylpyridiniumchlorid" (CPC)) bewährt. Hierbei handelt es sich um eine quaternäre Ammoniumverbindung mit bakterizider und fungizider Wirkung, welche unter anderem in Lutschtabletten zur Anwendung kommt. Nachteilig bei diesem Wirkstoff ist die Tatsache, daß bei hoher Dosierung und übermäßigem Verzehr Magen-Darm-Beschwerden, Atemnot sowie eine vermehrte Methämoglobinbildung, insbesondere bei Kindern, auftreten kann.

Des weiteren hat sich als topisches Antiseptikum bzw. Desinfektionsmittel für die Mund-, Hals- und Rachenschleimhaut auch 1,3-Bis(2-ethylhexyl)-hexahydro-5-methyl-5-pyridinamin (internationaler Freiname: "Hexetidin") bewährt, welches beispielsweise als Spray oder in Form von Spül- bzw. Gurgellösungen appliziert werden kann. Bei längerer Anwendung und starker Dosierung kann es auch hier zu Magen-Darm-Beschwerden und darüber hinaus zu Geschmacksirritationen kommen.

Des weiteren kommen Wirkstoffe natürlichen Ursprungs zum Einsatz, so z. B. sogenannte Schleimdrogen oder deren Extrakte, wie z. B. Isländisches Moos (*Lichen islandicus*)*.* Diese Wirkstoffe werden häufig im Rahmen von Monopräparaten eingesetzt. Dabei ist es vorrangig vorgesehen, die Wirkstoffe in fester, zum Lutschen geeigneter Form bereitzustellen, beispielsweise in Form einer festen Lutschtablette. Derartige Präparate bieten aber nicht immer den gewünschten Therapieerfolg.

Die EP 1 829 548 A1 betrifft die Verwendung von Extrakten aus *Pelargonium*-Arten in Kombination mit Plantagoextrakt zur topischen Prophylaxe oder Behandlung. Insbesondere kann die Behandlung in Form von Lutschpastillen zur topischen Behandlung von Mund- und Rachenraumentzündungen oder als Creme zur topischen Behandlung von Hauterkrankungen erfolgen.

Die EP 0 870 507 A1 betrifft eine antibakterielle Zusammensetzung, welche einen Kräuterextrakt und ein ätherisches Öl in einem definierten Verhältnis aufweisen soll. Das ätherische Öl soll eine antimikrobielle Wirkung aufweisen, wohingegen der Kräuterextrakt bzw. der Extrakt aus pflanzlichen Materialien eine deutlich geringere oder gar keine antimikrobielle Wirkung aufweisen soll. Spezielle Mischungen der beiden Komponenten sollen eine antimikrobielle Wirkung entfalten, welche die ihre Einzelkomponenten bei weitem übertrifft.

Die GB 2 093 696 A betrifft eine Zusammensetzung, welche zur Behandlung des Haars und/oder der Haut geeignet ist. Die Zusammensetzung enthält in einem wässrigen Medium pulverisierte Blumen oder Blüten mit einer Partikelgröße von nicht größer als 125 *µ*m in Gegenwart eines Klebstoffs, welcher es ermöglicht, die Homogenität der Zusammensetzung beizubehalten. Bei dem Klebstoff handelt es sich um ein Verdickungsmittel und/oder um eine Wasser-in-Öl- oder eine Öl-in-Wasser-Emulsion.

Der vorliegenden Erfindung liegt daher die Aufgabe zugrunde, eine insbesondere pharmazeutische Zusammensetzung bereitzustellen, welche sich zur effizienten topischen Behandlung von entzündlichen Erkrankungen des Mund- und Rachenraums (d. h. also von entzündlichen Erkrankungen des Hals-/Rachenraums und der Mundhöhle) sowie von Husten und Katharren der oberen Luftwege eignet und insbesondere die zuvor geschilderten Nachteile des Standes der Technik zumindest weitgehend vermeiden oder wenigstens abschwächen soll. Dabei soll insbesondere eine verbesserte Wirksamkeit gegenüber den zuvor genannten Indikationen bei einfacher und sicherer Applikation der Zusammensetzung erreicht werden. Zudem soll eine Zusammensetzung mit verbesserter Wirksamkeit bei gleichzeitig verringerten Nebenwirkungen bereitgestellt werden.

Zur Lösung der zuvor geschilderten Aufgabenstellung schlägt die vorliegende Erfindung - gemäß einem ersten Aspekt der vorliegenden Erfindung - eine Zusammensetzung, insbesondere pharmazeutische Zusammensetzung, gemäß Anspruch 1 vor. Weitere vorteilhafte Ausgestaltungen der erfindungsgemäßen Zusammensetzung sind Gegenstand der diesbezüglichen Unteransprüche.

Weiterer Gegenstand der vorliegenden Erfindung - gemäß einem weiteren Aspekt der vorliegenden Erfindung - ist die erfindungsgemäße Verpackungseinheit gemäß Anspruch 14, welche die Zusammensetzung nach der Erfindung, insbesondere in Form einer zur Einzeldosierung geeigneten Form, wie Lutschtabletten, enthält.

Schließlich ist weiterer Gegenstand der vorliegenden Erfindung - gemäß einem weiteren Aspekt der vorliegenden Erfindung - die erfindungsgemäße Verwendung der Zusammensetzung nach der Erfindung zur topischen Behandlung von entzündlichen Erkrankungen des Mund- und Rachenraums, Husten und Katharren der oberen Luftwege nach Anspruch 15.

Es versteht sich von selbst, daß Ausgestaltungen, Ausführungsformen, Vorteile und dergleichen, welche nachfolgend zu Zwecken der Vermeidung von Wiederholungen nur zu einem Erfindungsaspekt aufgeführt sind, selbstverständlich auch in bezug auf die übrigen Erfindungsaspekte entsprechend gelten.

Weiterhin gilt, daß die nachstehend genannten relativen bzw. prozentualen gewichtsbezogenen Mengenangaben im Rahmen der erfindungsgemäßen Zusammensetzung vom Fachmann derart auszuwählen sind, daß sie sich in der Summe unter Einbeziehung der Komponenten bzw. Inhaltsstoffe bzw. Zusatzstoffe bzw. Bestandteile bzw. Exzipienten, insbesondere wie nachfolgend definiert, stets um 100 Gew.-% ergänzen. Dies versteht sich für den Fachmann aber von selbst. Im übrigen gilt, daß der Fachmann anwendungsbezogen oder einzelfallbedingt von den nachfolgend angeführten Mengenangaben abweichen kann, ohne daß er den Rahmen der vorliegenden Erfindung verläßt.

Der Begriff "pharmazeutische Zubereitung" - synonym bisweilen auch als "pharmazeutische Zusammensetzung", "pharmazeutische Kombination" etc. bezeichnet - ist im Rahmen der vorliegenden Erfindung sehr breit zu verstehen und umfaßt jede Art von möglicher pharmazeutischer Zubereitung, Zusammensetzung oder Kombination, insbesondere Arzneimittel bzw. Pharmaka als solche, aber auch sogenannte Medizinprodukte, homöopathische Mittel und dergleichen.

Die Anmelderin hat nun überraschenderweise herausgefunden, daß das zuvor geschilderte Problem dadurch gelöst werden kann, daß man eine insbesondere pharmazeutische Zubereitung bzw. Zusammensetzung bzw. Kombination in einer zum Lutschen geeigneten Darreichungsform auf Basis einer Kombination von mindestens einer ersten Wirkstoffkomponente, welche mindestens eine Gerbstoffdroge bzw. deren Extrakt enthält, mit mindestens einer zweiten Wirkstoffkomponente, welche mindestens eine Schleimdroge bzw. deren Extrakt enthält, formuliert. Eine derartige Zusammensetzung eignet sich insbesondere zur Herstellung von insbesondere mehrphasigen Lutschtabletten, welche bei entzündlichen Erkrankungen des Mund- und Rachenraums sowie bei Husten und Katharren der oberen Luftwege zu einer raschen Linderung der Symptome, einhergehend mit einer beschleunigten Abheilung der Erkrankungen, führen. In diesem Zusammenhang hat die Anmelderin in völlig überraschender Weise herausgefunden, daß die Wirksamkeit der erfindungsgemäßen Zusammensetzung noch weiter gesteigert werden kann, wenn die erfindungsgemäße Zusammensetzung in Form einer Lutschtablette mit einer insbesondere festen Hülle, welche die erste Wirkstoffkomponente mit der Gerbstoffdroge enthält, und einem insbesondere flüssigen Kern, welcher die zweite Wirkstoffkomponente mit der Schleimdroge enthält, formuliert wird.

Gegenstand der vorliegenden Erfindung - gemäß einem ersten Aspekt der vorliegenden Erfindung - ist somit eine pharmazeutische Zusammensetzung in einer zum Lutschen geeigneten Darreichungsform zur topischen Behandlung von entzündlichen Erkrankungen des Mund- und Rachenraums, Husten und Katarrhen der oberen Luftwege, wobei die Zusammensetzung in pharmazeutisch wirksamen Mengen eine Kombination von
(a) 0,05 bis 30 Gew.-%, bezogen auf die pharmazeutische Zusammensetzung, mindestens einer ersten Wirkstoffkomponente, welche mindestens eine Gerbstoffdroge in Form eines Extraktes mit einem Droge/Extrakt-Verhältnis im Bereich von 1 : 0,1 bis 1 : 100 enthält, wobei die Gerbstoffdroge ausgewählt ist aus *Pelargonium,* Brombeere (*Rubus fructiosus*)*,* Eiche (*Quercus rubor* und/oder *Quercus petraea*), Gänsefingerkraut (*Potentilla anserina),* Gewürznelken (*Szygium aromaticum*), Heidelbeere *(Vaccinium myrtillus),* Myrrhe (*Commiphora molmol*)*,* Ratanhia (*Krameria triandra*)*,* Salbei (*Salvia triloba*)*,* Schlehdorn (*Prunus spinosa*)*,* Taubnessel (*Lamium album*)*,* Blutwurz (*Potentilla erecta*)*,* Erdbeere (*Fragaria vesca*)*,* Odermenningkraut (*Agrimonia eupatoria*)*,* Frauenmantelkraut (*Alchemilla xanthochlora*)*,* Rose (*Rosa gallica*), Wiesenknopf (*Sanguisorba officinalis*) und deren Kombinationen; mit
(b) 0,01 bis 20 Gew.-%, bezogen auf die pharmazeutische Zusammensetzung, mindestens einer zweiten Wirkstoffkomponente, welche mindestens eine Schleimdroge in Form eines Extraktes mit einem Droge/Extrakt-Verhältnis im Bereich von 0,4: 1 bis 15: 1 enthält, wobei die Schleimdroge ausgewählt ist aus Spitzwegerich *(Plantago lanceolata L.),* Isländischem Moos (*Lichen islandicus*)*,* Eibisch (*Althaea officinalis L.*), Malve (*Malva sylvestris L.* und *M*. *neglecta WALLR.*), Boxhornklee (*Trigonella foenum-graecum L.*)*,* Salep und Quitte (*Cydonia oblonga MILL.)* und deren Kombinationen;
umfaßt, wobei die Zusammensetzung mindestens eine erste Phase (A) und mindestens eine von der ersten Phase (A) umgebene zweite Phase (B) aufweist, wobei die erste Phase (A) mindestens die erste Wirkstoffkomponente (a) aufweist und die zweite Phase (B) mindestens die zweite Wirkstoffkomponente (b) aufweist, wobei die erste Phase (A) als Hülle und die zweite Phase (B) als Kern ausgebildet ist, wobei die erste Phase (A) in fester, beim Lutschen und/oder unter Speicheleinfluss auflösbarer Form vorliegt und wobei die zweite Phase (B) in flüssiger Form vorliegt, wobei die Phasen (A) und (B) die Wirkstoffkomponenten (a) und (b) beim Lutschen und/oder unter Speicheleinfluss zeitversetzt freisetzen, wobei die erste Wirkstoffkomponente (a) beim Lutschen und/oder unter Speicheleinfluss zeitlich vor der zweiten Wirkstoffkomponente (b) freigesetzt wird, wobei die Zusammensetzung die Wirkstoffkomponenten (a) und/oder (b) unabhängig voneinander jeweils in einer Matrix und/oder Masse auf Basis von Zuckern und/oder Zuckeraustauschstoffen enthält und wobei das gewichtsbezogene Verhältnis der festen ersten Phase (A) einerseits zu der flüssigen zweiten Phase (B) andererseits im Bereich von 50 - 98 : 50 - 2 liegt.

Die vorgenannte Kombination eignet sich insbesondere zur prophylaktischen und/oder kurativen, insbesondere symptomatischen prophylaktischen und/oder kurativen Behandlung der zuvor genannten Erkrankungen. Zudem eignet sich die vorgenannte Kombination insbesondere zur temporären alleinigen oder unterstützenden Behandlung von Entzündungen des Mund- und Rachenraums, beispielsweise im Rahmen von Erkältungserkrankungen und dergleichen. Durch die Verwendung natürlich basierter Wirkstoffe auf Basis von mindestens einer Gerbstoffdroge und mindestens einer Schleimdroge resultiert bei gleichzeitig hoher Wirksamkeit der erfindungsgemäßen Zusammensetzung eine gute Verträglichkeit einhergehend mit einer weitestgehenden Verhinderung von Nebenwirkungen. Das Wirkprinzip der erfindungsgemäßen Zusammensetzung basiert dabei auf rein physikalischen bzw. physikochemischen Prozessen, so daß keine Rezeptorwechselwirkung im pharmakologischen Sinne vorliegt. Das grundlegende Prinzip der vorliegenden Erfindung beruht dabei auf einer gezielten Kombination von Wirkstoffkomponenten auf Basis von Gerbstoffdrogen einerseits und Wirkstoffkomponenten auf Basis von Schleimdrogen andererseits, wobei die Anmelderin in völlig überraschender Weise herausgefunden hat, daß die Wirksamkeit der Kombination von Gerbstoffdrogen einerseits und Schleimdrogen andererseits in bezug auf die zuvor angeführten Erkrankungen noch weiter erhöht werden kann, wenn die jeweiligen Wirkstoffkomponenten zeitversetzt verabreicht werden, wie nachfolgend noch ausführlich erläutert.

Die hervorragende Wirksamkeit der erfindungsgemäßen Kombination beruht auf der speziellen Abstimmung der Wirkweise von Gerbstoffdroge und Schleimdroge, wobei sich die Einzelwirkungen in synergistischer Weise ergänzen:
Der Wirkmechanismus der im Rahmen der vorliegenden Erfindung eingesetzten Gerbstoffdrogen kann - ohne sich auf diese Theorie beschränken zu wollen - derart verstanden werden, daß die Gerbstoffdrogen bei Verabreichung der erfindungsgemäßen Zusammensetzung im Mund bzw. im Rachen, insbesondere bei deren Freisetzung, zunächst die Proteine in der Mund- bzw. Rachenschleimhaut vernetzen, so daß gewissermaßen eine oberflächliche Verdichtung bzw. eine Bildung einer schützenden Membran aus vernetzten Proteinen resultiert. Auf diese Weise wird Infektionen vorgebeugt bzw. vorhandene Infektionen werden abgeschwächt. In diesem Zusammenhang weisen die erfindungsgemäß eingesetzten Gerbstoffdrogen auch eine adstringierende Wirkung sowie antivirale und antibakterielle Eigenschaften auf, so daß das Wachstum von Bakterien bzw. die Vermehrung von Viren, die sich beispielsweise auf der Schleimhaut angesiedelt haben, wirkungsvoll reduziert bzw. unterbunden wird. Dadurch werden auch Schmerzen und Wundsekretion vermindert, Entzündungen gehemmt sowie kapillare Blutungen gestillt.

Was die Wirkweise der erfindungsgemäß eingesetzten Schleimdrogen anbelangt, so kann diese - ohne sich auf diese Theorie beschränken zu wollen - derart verstanden werden, daß die Schleimdrogen bei ihrer Freisetzung im Rahmen der Verabreichung der erfindungsgemäßen Zusammensetzung einen schützenden Film über die geschädigten Schleimhäute ausbilden, wobei diese Filmbildung zu einem weiterführenden Schutz der Schleimhäute, einhergehend mit einer schnelleren Abheilung der Entzündung, führt.

In diesem Zusammenhang hat die Anmelderin zudem in überraschender Weise herausgefunden, daß durch eine zeitlich versetzte Freisetzung im Rahmen der Applikation der erfindungsgemäßen Zusammensetzung, wonach in zeitlicher Abfolge zunächst die Gerbstoffdroge und anschließend die Schleimdroge freigesetzt wird, besonders gute Ergebnisse hinsichtlich der Behandlung der zuvor beschriebenen Erkrankungen resultieren. Ohne sich auf diese Theorie festlegen zu wollen, kann die überraschend gefundene verbesserte Wirkung auf Basis einer zeitlich versetzten Freisetzung von Gerbstoffdroge einerseits und Schleimdroge andererseits dahingehend erklärt werden, daß zunächst auf Basis der Gerbstoffe eine Vernetzung der entzündlichen Proteine an der Schleimhaut erfolgt, woraufhin die Schleimdrogen eine schützende Schleimschicht über diese legen können. Dabei vernetzen die Gerbstoffe, wie zuvor geschildert, zunächst die entzündeten Proteine der (Mund-)Schleimhaut; anschließend können sich die Schleimstoffe an die Gerbstoffe haften, wobei die Vernetzung der Schleimstoffe verbessert ist, insbesondere da noch nicht vollständig abgefangene Gerbstoffe dazu führen, daß die Schleimstoffe besser an dem entzündeten Gewebe anhaften und dadurch in besonders vorteilhafter Weise ihre schützende Funktion ausüben können.

Was die erfindungsgemäße Zusammensetzung somit anbelangt, so ist diese als Mehrphasensystem, insbesondere mindestens als Zweiphasensystem, vorzugsweise als Zweiphasensystem, ausgebildet, wobei die jeweiligen Phasen beim Lutschen und/oder unter Speicheleinfluß zeitversetzt aufgelöst und/oder freigesetzt werden bzw. wobei die jeweiligen Phasen die in den jeweiligen Phasen enthaltenden Wirkstoffe und/oder Inhaltsstoffe beim Lutschen und/oder unter Speicheleinfluß zeitversetzt freisetzen.

Der Begriff "Phase" wie er im Rahmen der vorliegenden Erfindung verwendet wird, bezieht sich insbesondere auf eine differenzierte Ausbildung der erfindungsgemäßen Zusammensetzung hinsichtlich ihrer Darreichungs- bzw. Applikationsform, beispielsweise in Form von Lutschtabletten. Diesbezüglich kann eine Phase gewissermaßen einen Abschnitt bzw. Bereich der Darreichungs- bzw. Applikationsform der erfindungsgemäßen Zusammensetzung darstellen, welcher sich beispielsweise hinsichtlich seiner Zusammensetzung und/oder seines Aggregatzustandes vor der Anwendung von einer anderen Phase - also einem anderen Bereich bzw. Abschnitt der Zusammensetzung - unterscheidet. So kann, worauf im nachfolgenden noch eingegangen wird, die erfindungsgemäße Zusammensetzung beispielsweise eine äußere Phase in fester Form aufweisen, welche nach Art einer Hülle eine weitere innere Phase, beispielsweise in Form eines flüssigen Kerns, umgeben bzw. umschließen kann.

Im Rahmen der vorliegenden Erfindung ist es vorteilhaft, wenn die Wirkstoffkomponenten (A) und (B) in voneinander verschiedenen Phasen enthalten sind. Auf diese Weise kann beispielsweise bei der Applikation die zeitversetzte Freisetzung der ersten Wirkstoffkomponente (A) einerseits und der zweiten Wirkstoffkomponente (B) andererseits erreicht werden.

Mit anderen Worten ist die erfindungsgemäße Zusammensetzung derart ausgebildet, daß die erfindungsgemäße Zusammensetzung bzw. Zubereitung mehrere Phasen, insbesondere mindestens zwei Phasen, vorzugsweise zwei Phasen, umfaßt. Dabei kann es im Rahmen der vorliegenden Erfindung vorgesehen sein, daß die jeweiligen Phasen jeweils mindestens eine Wirkstoffkomponente enthalten, beispielsweise kann eine erste Phase mindestens die erste Wirkstoffkomponente (a), welche mindestens eine Gerbstoffdroge bzw. deren Extrakt enthält, umfassen. In diesem Zusammenhang kann eine zweite Phase die zweite Wirkstoffkomponente (b) mit mindestens einer Schleimdroge bzw. deren Extrakt enthalten. Diesbezüglich kann es erfindungsgemäß - gemäß einer bevorzugten Ausführungsform - vorgesehen sein, daß bei Anwendung der erfindungsgemäßen Zusammensetzung, insbesondere beim Lutschen, im zeitlichen Verlauf zunächst die erste Wirkstoffkomponente (a) aus der ersten Phase und anschließend bzw. zeitlich nachfolgend die zweite Wirkstoffkomponente (b) aus der zweiten Phase freigesetzt wird. Grundsätzlich kann der Begriff "zeitversetzt", wie er im Rahmen der vorliegenden Erfindung verstanden wird, auch eine gewisse Überlappung bzw. einen gewissen Übergang der Freisetzung der Wirk- bzw. Inhaltsstoffe umfassen.

Erfindungsgemäß ist es vorgesehen, daß die erfindungsgemäße Zusammensetzung mindestens eine erste Phase (A) und mindestens eine zweite Phase (B) umfaßt, wobei die erste Phase (A) mindestens die erste Wirkstoffkomponente (a) und die zweite Phase (B) mindestens die zweite Wirkstoffkomponente (b) enthält. Diesbezüglich können die Phasen (A) und (B) die Wirkstoffkomponenten (a) und (b) beim Lutschen und/oder unter Speicheleinfluß zeitversetzt freisetzen. In diesem Zusammenhang sollte die erfindungsgemäße Zusammensetzung dergestalt sein, daß die erste Wirkstoffkomponente (a) zumindest im wesentlichen ausschließlich in der Phase (A) enthalten ist und die zweite Wirkstoffkomponente (b) zumindest im wesentlichen ausschließlich in der Phase (B) enthalten ist.

Gemäß einer erfindungsgemäß besonders bevorzugten Ausführungsform sollte die erste Phase (A) die zweite Phase (B) umgeben bzw. diese zumindest im wesentlichen umschließen. Diesbezüglich ist die erste Phase (A) gewissermaßen als Hülle ("Mantel") und die zweite Phase (B) gewissermaßen als Kern ("Füllung") ausgebildet. Es resultiert somit gemäß einer erfindungsgemäß besonders bevorzugten Ausführungsform ein Kern/Hülle-System bzw. ein Mantel/Füllung-System, wobei die Wirkstoffkomponente (b) im Kern enthalten und die erste Wirkstoffkomponente (a) in der Hülle enthalten ist. Beim Lutschen bzw. unter Speicheleinfluß resultiert folglich unter Auflösung der Hülle und somit der Phase (A) zunächst eine Freisetzung der ersten Wirkstoffkomponente (a) und nachfolgend nach Auflösung der Hülle eine Freisetzung der zweiten Wirkstoffkomponente (b) aus dem Kern bzw. aus der Phase (B). Es wird somit zunächst die Gerbstoffdroge und anschließend die Schleimdroge freigesetzt, was unter Bezugnahme der obigen Ausführungen hinsichtlich der Wirksamkeit von besonderem Vorteil ist.

Im Rahmen der vorliegenden Erfindung ist es also gemäß einer besonders bevorzugten Ausführungsform vorgesehen, daß die erste Wirkstoffkomponente (a) beim Lutschen und/oder unter Speicheleinfluß zeitlich vor der zweiten Wirkstoffkomponente (b) freigesetzt wird.

Was die Zusammensetzung nach der Erfindung weiterhin anbelangt, so kann die Zusammensetzung mindestens eine Hülle und mindestens einen von der Hülle umgebenen Kern aufweisen. Dabei kann die Hülle die erste Wirkstoffkomponente (a), insbesondere die mindestens eine Gerbstoffdroge und/oder deren Extrakt, enthalten, und der Kern kann die zweite Wirkstoffkomponente (b), insbesondere die mindestens eine Schleimdroge und/oder deren Extrakt, enthalten. In diesem Zusammenhang kann die erste Phase (A) als Hülle ausgebildet sein, und die zweite Phase (B) kann als Kern ausgebildet sein.

Gemäß der vorliegenden Erfindung kann es zudem vorgesehen sein, daß die erfindungsgemäße Zusammensetzung mindestens zwei Phasen aufweist, insbesondere in Form mindestens einer äußeren Phase und mindestens einer inneren Phase, wobei die äußere Phase die Wirkstoffkomponente (a), insbesondere die Gerbstoffdroge und/oder deren Extrakt, und die innere Phase die zweite Wirkstoffkomponente (b), insbesondere die Schleimdroge und/oder deren Extrakt, umfaßt, insbesondere wobei beim Lutschen in zeitlicher Abfolge zunächst die Wirkstoffkomponente (a) und nachfolgend die Wirkstoffkomponente (b) freigesetzt wird.

Die Anmelderin hat zudem in völlig überraschender Weise herausgefunden, daß sich die Wirksamkeit der erfindungsgemäßen Zusammensetzung auf Basis der gezielten Kombination von Gerbstoffdrogen einerseits mit Schleimdrogen andererseits und deren zeitlich versetzten Freisetzung noch bessere Ergebnisse erhalten lassen, wenn - in bezug auf die anwendungsfertige Zusammensetzung nach der Erfindung - die erste Wirkstoffkomponente (a) und/oder die erste Phase (A) und/oder die äußere Phase und/oder die Hülle in fester und/oder beim Lutschen auflösbarer Form vorliegt bzw. vorliegen und, unabhängig von der ersten Phase (A), die zweite Wirkstoffkomponente (b) bzw. die zweite Phase (B) bzw. die innere Phase bzw. der Kern in flüssiger Form vorliegt bzw. vorliegen.

Die seitens der Anmelderin durchgeführten Anwendungsbeobachtungen bzw. -studien zeigen, daß durch den gezielten Einsatz eines flüssigen Kerns, welcher die Wirkstoffkomponente (b) und somit die Schleimdroge enthält, die Wirksamkeit der erfindungsgemäßen Zusammensetzung in bezug auf die genannten Indikationen signifikant verbessert wird. Ohne sich auf eine Theorie festlegen zu wollen, kann dies dadurch erklärt werden, daß aufgrund der Bereitstellung des Kerns in flüssiger Form bei dessen Freisetzung, also nach Auflösung der festen Hülle bzw. der festen ersten Phase (A) mit der Wirkstoffkomponente (a), eine bessere räumliche Verteilung der zweiten Wirkstoffkomponente (b) und somit der Schleimdrogen im Mund- und Rachenraum resultiert, was zu einer verbesserten Ausbildung der durch die Schleimdroge ausgebildeten Schutzschicht auf der (Mund-)Schleimhaut führt. Zudem wird eine Verabreichung der Zusammensetzung nach der Erfindung auf Basis einer spezifischen Kombination einer festen Hülle und eines flüssigen Kerns als angenehm empfunden, und der beim Lutschen stattfindende Phasenwechsel von der festen Hülle zu dem flüssigen Kern ruft beim Anwender zudem auf organoleptischer Ebene einen Hinweis auf die Doppel- bzw. Zweiphasenwirkung der erfindungsgemäßen Zusammensetzung hervor. Zudem resultiert eine kürzere Anwendungsdauer, da der flüssige Kern beim Lutschen bzw. Speichelkontakt nicht erst aufgelöst werden muß.

Zudem hat es sich als vorteilhaft erwiesen, wenn die zweite Wirkstoffkomponente (b) und/oder die zweite Phase (B) und/oder der Kern in einer flüssigen, vorzugsweise dickflüssigen, insbesondere sirupösen, bis hin zu einer pastösen oder gar gelartigen Form ausgebildet ist. Dabei kann die dynamische Viskosität bei Raumtemperatur (20° C) und Umgebungsdruck (Atmosphärendruck) der zweiten Wirkstoffkomponente (b) und/oder der zweiten Phase (B) und/oder des Kerns im Bereich von 1 bis 1.000 mPa·s, insbesondere 5 bis 800 mPa·s, vorzugsweise 10 bis 500 mPa·s, bevorzugt 15 bis 300 mPa·s, besonders bevorzugt 20 bis 200 mPa·s, liegen. Hierdurch resultiert eine weitere Optimierung hinsichtlich der Freisetzung der Wirkstoffkomponente (b), da gewissermaßen die Matrixstruktur, in welcher die Wirkstoffkomponente (b) eingelagert ist, weiter optimiert ist und die Verteilung aufgrund der speziellen Einstellung der Viskosität im Mund weiter verbessert ist.

In bezug auf die nachfolgenden Ausführungen zu der ersten Wirkstoffkomponente (a) und der zweiten Wirkstoffkomponente (b) ist anzuführen, daß die erste Wirkstoffkomponente (a) die Gerbstoffdroge(n) enthält oder aus dieser bzw. diesen gebildet ist. Entsprechendes gilt für die zweite Wirkstoffkomponente (b), welche gleichermaßen die Schleimdroge(n) enthält oder aus dieser bzw. diesen gebildet ist.

Was die erste Wirkstoffkomponente (a) anbelangt, so ist die erste Wirkstoffkomponente (a), d.h. Gerbstoffdroge, in Form eines Extraktes, insbesondere Flüssigextraktes, vorzugsweise in Form einer Urtinktur, zugesetzt diesbezüglich sollte der Extrakt ausgehend von einem wäßrigen, alkoholischen oder wäßrig-alkoholischen Extrakt, bevorzugt einem wäßrig-alkoholischen Extrakt, erhalten sein.

Im allgemeinen - und somit auch in bezug auf die zweite Wirkstoffkomponente (b) - bietet die Verwendung eines Extraktes einer Droge gegenüber der Verwendung der Droge als solcher den entscheidenden Vorteil, daß hohe Konzentrationen der Droge zur Anwendung kommen, so daß eine besonders gute therapeutische Wirkung erzielt wird. Zudem können hierdurch Verunreinigungen minimiert werden.

In diesem Zusammenhang werden besonders gute therapeutische Ergebnisse erzielt, wenn die erste Wirkstoffkomponente (a), insbesondere die Gerbstoffdroge, in Form eines Extraktes, insbesondere Flüssigextraktes, vorzugsweise in Form einer Urtinktur, mit einem Droge/Extrakt-Verhältnis im Bereich von 1 : 0,1 bis 1 : 100, insbesondere 1 : 0,5 bis 1 : 50, vorzugsweise 1 : 1 bis 1 : 20, bevorzugt 1 : 5 bis 1 : 15, zugesetzt ist.

Was die Menge der ersten Wirkstoffkomponente (a), insbesondere der Gerbstoffdroge in der erfindungsgemäßen Zusammensetzung bzw. Kombination anbelangt, so kann diese Menge in weiten Bereichen variieren. Im allgemeinen enthält die erfindungsgemäße Zusammensetzung die erste Wirkstoffkomponente (a), insbesondere die Gerbstoffdroge und/oder deren Extrakt, in Mengen von 0,05 bis 30 Gew.-%, insbesondere 0,1 bis 20 Gew.-%, vorzugsweise 0,2 bis 10 Gew.-%, bevorzugt 0,5 bis 5 Gew.-%, bezogen auf die pharmazeutische Zusammensetzung. Dennoch kann es anwendungsbezogen gegebenenfalls vorteilhaft oder erforderlich sein, von den vorgenannten Mengenangaben abzuweichen.

Erfindungsgemäß ist die Gerbstoffdroge ausgewählt aus *Pelargonium,* insbesondere *Pelargonium sidoides* und/oder *Pelargonium reniforme,* Brombeere (*Rubus fructiosus*)*,* Eiche (*Quercus rubor* und/oder *Quercus petraea*)*,* Gänsefingerkraut (*Potentilla anserina*)*,* Gewürznelken *(Szygium aromaticum*)*,* Heidelbeere (*Vaccinium myrtillus*), Myrrhe (*Commiphora molmol),* Ratanhia (*Krameria triandra*)*,* Salbei (*Salvia triloba*)*,* Schlehdorn *(Prunus spinosa*)*,* Taubnessel (*Lamium album*)*,* Blutwurz (*Potentilla erecta*)*,* Erdbeere (*Fragaria vesca*)*,* Odermenningkraut (*Agrimonia eupatoria*)*,* Frauenmantelkraut (*Alchemilla xanthochlora*)*,* Rose (*Rosa gallica*)*,* Wiesenknopf (*Sanguisorba officinalis*) und deren Kombinationen, vorzugsweise aus *Pelargonium,* insbesondere *Pelargonium sidoides* und/oder *Pelargonium reniforme.*

Im Rahmen der vorliegenden Erfindung ist es besonders vorteilhaft, wenn die erste Wirkstoffkomponente (a), insbesondere die Gerbstoffdroge, *Pelargonium,* insbesondere *Pelargonium sidoides* und/oder *Pelargonium reniforme,* ist bzw. diese umfaßt, insbesondere in Form eines Extraktes, vorzugsweise Flüssigextraktes, bevorzugt in Form einer Urtinktur. Bei dem bevorzugten Bestandteil des Pelargonium-Extraktes handelt es sich beispielsweise um einen wäßrig-ethanolischen Auszug aus der Wurzel von *Pelargonium sidoides* bzw. *Pelargonium reniforme.* Beispielsweise kann im Rahmen der vorliegenden Erfindung eine Urtinktur von *Pelargonium sidoides* eingesetzt werden, bei welcher die eingesetzte Droge *Pelargonium sidoides radix* ist. Diese Urtinktur kann ein Verhältnis von Droge zu Urtinktur von etwa 1 : 8 - 9 und ein Verhältnis von Droge zu Auszugsmittel von etwa 1: 10 aufweisen. Als diesbezügliches Auszugsmittel kann beispielsweise Ethanol 15 % (m/m) dienen, z. B. hergestellt aus Ethanol 96 % Ph. Eur. und gereinigtem Wasser Ph. Eur.). Bei dieser Urtinktur handelt es sich um eine klare, braune Flüssigkeit. Der Trockenrückstand dieser Urtinktur kann nach Ph. Eur. etwa 1 % betragen.

Die zuvor angeführten Spezies weisen Gerbstoffe auf, welche im Rahmen der vorliegenden Erfindung, wie zuvor beschrieben, mit den entzündeten Proteinen der (Mund-) und Rachenschleimhaut interagieren und neben einer adstringierenden Wirkung auch schmerzlindernd, blutstillend, sekretionshemmend, schleimhautschützend, keimhemmend, bakteriozid, fungizid und entzündungshemmend sind. Die Adstringenswirkung beruht insbesondere auf einer Wechselwirkung mit Eiweißmolekülen der Collagenfasern der obersten Gewebeschichten der Schleimhäute. Diese Wirkung zeigen insbesondere Gerbstoffe mit einem Molekulargewicht zwischen 500 und 3.000 und mit 1 bis 2 Hydroxylgruppen pro 100 Masseeinheiten. Aufgrund ihrer chemischen Eigenschaften unterteilt man die Stoffklasse der Gerbstoffe in kondensierte und hydrolysierbare Gerbstoffe. So zählen zu den kondensierten Gerbstoffen z. B. Catechingerbstoffe und die Proanthocyanidine.

Bausteine der kondensierten Catechingerbstoffe sind die Flavanole (+)-Catechin und (+)-Epicatechin. Es treten auch Mischkondensate auf, die zusätzlich Leukoanthocyanidine enthalten und als Proanthocyanidine bezeichnet werden. Die hydrolysierbaren Gerbstoffe lassen sich durch Hydrolyse in Zucker und Gallussäure bzw. Ellagsäure (Hexahydroxydiphensäure) spalten. Daher unterscheidet man Gallotannine und Ellagitannine. Typischer Vertreter ist das "Tannin" (Hexagalloylglucose) oder die Digallussäure. Häufig treten auch Mischverbindungen auf, die bei Hydrolyse sowohl Gallussäure als auch Catechin ergeben, wie z. B. Epicatechingallate.

In bezug auf die erfindungsgemäß bevorzugt eingesetzten *Pelargonium*-Spezies*,* insbesondere *Pelargonium sidoides* und/oder *Pelargonium reniforme,* beträgt der Gesamtgerbstoffgehalt in diesen Spezies in den Wurzeln etwa 9 % der Trockenmasse. Dabei ist der Gehalt an hydrolysierbaren Gerbstoffen sehr gering, da die gefundenen Werte für Gallussäure kleiner als 0,01 % in der Trockenmasse und für Gallussäuremethylester ca. 0,02 % in der Trockenmasse betragen. Es dominieren demnach Catechingerbstoffe, für die als Monomere meist Catechin und Gallocatechin und in geringem Umfang auch Afzelechin nachgewiesen wurden. Diese Monomeren liegen in einem komplex zusammengesetzten Gemisch polymerer Strukturen von mindestens acht Flavanoleinheiten vor, deren Molekulargewicht ca. 2.300 beträgt und damit noch im Bereich einer adstringierenden Wirkung liegt.

Was den Wirkmechanismus hinsichtlich der Adsorption von Gerbstoffen an Proteinen anbelangt, so kann dieser insofern verstanden werden, daß - ohne sich auf diese Theorie beschränken zu wollen - beim Kontakt von Gerbstoffen mit Eiweißmolekülen zunächst eine Adsorption der Substanz an die offenen bzw. amorphen, nichtquasikristallinen Abschnitte der Eiweißmoleküle erfolgt. Für geringe Konzentrationen von Adstringens bzw. Gerbstoff im Vergleich zur Menge an Eiweiß mißt man eine lineare Abhängigkeit der Adsorption von der Konzentration. Dieser Sachverhalt einer linearen Beziehung zwischen Gerbstoffadsorption und Konzentration wird in der Analytik der Gerbstoffe genutzt. Experimentell wird durch einen hohen Überschuß an Hautpulver (Proteine) der Gerbstoff vollständig gebunden und dieses durch die Differenz der optischen Extinktion einer Farbreaktion vor und nach der Adsorption dargestellt. Bei weiterer Zugabe von Gerbstoff strebt der Adsorptionswert einem Grenzwert zu. Die hieraus resultierende Kurve (Adsorption gegen Konzentration) ist völlig verschieden von einer pharmakologischen Dosis/Wirkung-Beziehung. Die vorliegenden Verhältnisse sind aus der physikalischen Chemie bekannt und werden üblicherweise durch die Langmuir'sche Gleichung für Adsorptionsisothermen beschrieben. Das Langmuir'sche Gesetz der Adsorption kann als Funktion der adsorbierten Menge in Abhängigkeit von der Konzentration dargestellt werden. Der hierdurch beschriebene Sättigungswert resultiert aus der Ausbildung von sogenannten "Monolayern" und damit einer Sättigung der verfügbaren Oberfläche der Eiweißmoleküle mit dem Gerbstoff. Somit wird die Bildung von "Monolayern" durch Gerbstoffe auf Eiweißmolekülen und Vernetzung von Proteinen durch diese Gerbstoffschicht beschrieben.

Von Adstringenswirkung spricht man, wenn der Gerbstoff die Eiweißmoleküle (z. B. in Collagenfasern) durch weniger stabile Ionen- oder Wasserstoffbrückenbindungen vernetzt. Diese Vorgänge sind Grundlage der Wirkung der erfindungsgemäßen Zusammensetzung auf die Schleimhäute im Mund, da relativ wenig Gerbstoffe und eine große Eiweißmenge vorliegt. Aufgrund der relativ geringen Menge an vorhandenen Gerbstoffen werden diese an die Mundschleimhaut gebunden, können aber nicht die Epithelbarriere durchdringen und werden somit auch nicht resorbiert.

Zusammenfassend kann somit festgestellt werden, daß die insbesondere aus *Pelargonium*-Extrakt der erfindungsgemäßen Zusammensetzung freigesetzten Gerbstoffe die Proteine der entzündeten Schleimhaut in Mund und Rachen aufgrund ihrer adstringierenden Eigenschaften vernetzen, wobei es sich hierbei um physikochemische bzw. physikalische Wirkmechanismen der Adsorption und Ionenbindungen handelt, welche definitionsgemäß nicht zu pharmakologischen Wirkungen gezählt werden. Aufgrund des Wirkmechanismus der oberflächlichen Adsorption werden die Gerbstoffe im Mundraum zumindest im wesentlichen auch nicht resorbiert.

Was die erfindungsgemäß bevorzugt eingesetzten *Pelargonium*-Spezies betrifft, so stammen diese auch als Kap-Pelargonie bezeichnete Pflanze ursprünglich aus Südafrika. In Südafrika werden neben *Pelargonium sidoides* viele ganz ähnlich aussehende *Pelargonium*-Spezies als traditionelle Arzneipflanzen und auch als Lebensmittel sowie für andere Zwecke (wie Parfüme) verwendet. Wegen der bekannten Ungiftigkeit werden Blätter und Stiele aller Arten gerne als "*Bush Food Snack"* verzehrt, aber auch die Wurzeln als Gemüse für die Vorratshaltung geerntet.

Was die in der zweiten Wirkstoffkomponente (b) enthaltene Schleimdroge anbelangt, so ist diese in Form eines Extraktes, insbesondere eines Trockenextraktes, zugesetzt. Diesbezüglich sollte der Extrakt ausgehend von einem wäßrigen, alkoholischen oder wäßrig-alkoholischen Extrakt, bevorzugt einem wäßrigen Extrakt, erhalten sein. Sofern die Wirkstoffkomponente (b), insbesondere die Schleimdroge, in Form eines Trockenextraktes eingesetzt wird, sollte dieser in bevorzugter Weise zumindest im wesentlichen wasserlöslich sein.

Das Droge/Extrakt-Verhältnis kann in weiten Bereichen variieren. Wenn die zweite Wirkstoffkomponente (b), insbesondere die Schleimdroge, in Form eines Extraktes, vorzugsweise eines Trockenextraktes eingesetzt ist, sollte insbesondere ein Droge/Extrakt-Verhältnis im Bereich von im Bereich von 0,4 : 1 bis 15 : 1, insbesondere im Bereich von 1 : 1 bis 10 : 1, bevorzugt im Bereich von 5 : 1 bis 8 : 1, gewählt werden.

Was die Menge an zweiter Wirkstoffkomponente (b), insbesondere an Schleimdroge bzw. deren Extrakt, anbelangt, so kann diese Menge in weiten Bereichen variieren. Im allgemeinen enthält die erfindungsgemäße Zusammensetzung die zweite Wirkstoffkomponente (b), insbesondere die Schleimdroge und/oder deren Extrakt, in Mengen von 0,01 bis 20 Gew.-%, insbesondere 0,05 bis 10 Gew.-%, vorzugsweise 0,05 bis 5 Gew.-%, bevorzugt 0,08 bis 2 Gew.-%, bezogen auf die pharmazeutische Zusammensetzung.

Erfindungsgemäß eingesetzte Schleimdrogen, welche in der zweiten Wirkstoffkomponente (b) enthalten sind, sind ausgewählt aus Spitzwegerich *(Plantago lanceolata L.),* Isländischem Moos (*Lichen islandicus*)*,* Eibisch (*Althaea officinalis L*.)*,* Malve (*Malva sylvestris L.* und *M*. *neglecta WALLR*.), Boxhornklee (*Trigonella foenum-graecum L*.)*,* Salep und Quitte (*Cydonia oblonga MILL.*) und deren Kombinationen, vorzugsweise Spitzwegerich *(Plantago lanceolata L.).*

Erfindungsgemäß bevorzugt ist eine Ausführungsform, wonach die zweite Wirkstoffkomponente (b), insbesondere die Schleimdroge, Spitzwegerich (*Plantago lanceolata L*.) ist oder diesen umfaßt, insbesondere in Form eines Extraktes, insbesondere eines Trockenextraktes, insbesondere wobei der Extrakt ausgehend von einem wäßrigen, alkoholischen oder wäßrig-alkoholischen Extrakt, bevorzugt einem wäßrigen Extrakt, erhältlich ist. Denn diese Schleimdroge liefert im Rahmen der vorliegenden Erfindung die besten Ergebnisse.

Schleime sind Heteropolysaccharide mit Molekulargewichten von ca. 50.000 bis 2.000.000, die durch Extraktion mit heißem oder kaltem Wasser aus der Droge gewonnen werden. Die hochviskosen Lösungen sind im Gegensatz zu den Gummen nicht klebrig. Man unterscheidet saure und neutrale Schleime. Je nach ihrer Zuckerzusammensetzung lassen sie sich in Glucomannane oder Mannane, in Galactomannane, Xylane oder Rhamnogalacturonane einteilen. Nach ihrer Lokalisierung in der Pflanze kann man Vakuolenschleime und Membranschleime unterscheiden. Als Prototyp für einen sauren Pflanzenschleim kann der Schleim der Eibischwurzel angesehen werden. Er enthält L-Rhamnose, D-Galactose, D-Galacturonsäure und D-Glucuronsäure im molaren Verhältnis von ungefähr 3 : 2 : 3 : 3. Der am einfachsten gebaute Teil des Polysaccharids besteht aus sich wiederholenden Undecasaccharid-Untereinheiten.

Der Spitzwegerich (*Plantago lanceolata L*.) ist in ganz Europa sowie in Nord- und Mittelasien verbreitet. Die Droge stammt von wild wachsenden Pflanzen und Kulturen vor allem Südosteuropas. Die getrockneten, oliv- bis bräunlichgrün gefärbten, lanzettlichen Blattspreiten mit etwa drei bis sieben parallel verlaufenden Nerven können verwendet werden. Die Herba-Droge besteht aus den getrockneten Blättern, Stengeln und ganzen Blüten. Neben dem Schleim enthält der Spitzwegerich als weitere Inhaltsstoffe Tannine, das Iridoidglykosid Aucubin (1,9 bis 2,4 %), das für die Dunkelfärbung einer nicht sorgfältig getrockneten Droge verantwortlich ist und zur Identitätsprüfung herangezogen wird, ferner das Senföl Sulforaphen. Der Spitzwegerich, insbesondere das Spitzwegerichblätter-Kraut, findet insbesondere Anwendung in Form von Tees und Sirupen bei Entzündungen des Mund- und Rachenraums.

Isländisches Moos ist der deutsche Name für *Lichen islandicus* oder *Cetraria is-landica* (*Parmeliaceae*), einer Flechte - entgegen dem deutschen Namen kein Moos -, die aus nördlichen Ländern, wie beispielsweise von Island, Norwegen und Schweden, exportiert wird. Isländisches Moos im eigentlichen Sinne besteht aus dem getrockneten Thallus von *Cetraria islandica* sowie dessen Zubereitungen. Die Droge enthält unter anderem Schleimstoffe und Bitterstoffe sowie bitterschmeckende Flechtensäuren. Aus der gepulverten Flechte lösen sich etwa 60 Gew.-% beim Kochen mit stark verdünnter Natriumhydrogencarbonatlösung, und beim Abkühlen der Lösung entsteht eine Gallerte. Der Extrakt besteht aus einem Polysaccharidgemisch von Lichenin und Isolichenin, einer Reihe von bitterschmeckenden Flechtensäuren (Fumarprotocetrar-, Protocetrar- und Cetrarsäure) sowie Protolichesterinsäure, die sich bei der Aufarbeitung in Lichesterinsäure umwandelt, und Usninsäure als antibiotisch wirkendem Flechtenfarbstoff. Als Schleimdroge besitzt Isländisches Moos reizlindernde Eigenschaften, es wirkt ebenso antimikrobiell. Der Bitterstoffgehalt begründet seine Anwendung bei Appetitlosigkeit. Isländisches Moos wird beispielsweise bei Katarrhen und Durchfall, als Bittertonikum, äußerlich bei schlecht heilenden Wunden, bei kosmetischen Präparaten, in Haarfixiermitteln, als Zusatz zu Biskuitmehl und dergleichen, bei Appetitlosigkeit sowie bei Schleimhautreizungen im Mund- und Rachenraum angewandt. Ein Mund- und Rachendesinfizienz auf Basis von Isländischem Moos wirkt reizlindernd bei trockenem Reizhusten. Das Kaltmazerat und andere bitterschmeckende Zubereitungen der Droge werden zur Behebung der Sub- oder Antacidität eingesetzt. Als Darreichungsformen werden je nach Anwendungsgebiet die zerkleinerte Droge für Aufgüsse sowie andere galenische Zubereitungen bzw. die zerkleinerte Droge vorzugsweise für Kaltmazerate sowie andere bitterschmeckende Zubereitungen zum Einnehmen angewandt. Für weitergehende Einzelheiten zu Isländischem Moos kann beispielsweise auf die folgende Literaturstellen verwiesen werden: Römpp Chemie-Lexikon, 9. Auflage, Band 3, Georg Thieme Verlag, Stuttgart/New York, 1990, Seiten 2055/2056, Stichwort: "Isländisches Moos"; HagerROM 2001, Springer Verlag, Heidelberg, Stichworte: "Cetraria", "Cetraria ericetorum OPIZ", "Cetraria islandica (L.) ACHARIUS" und "Lichen islandicus (Isländisches Moos)"; Monographie "Liehen islandicus (Isländisches Moos)", Bundesanzeiger Nr. 43 vom 2. März 1989; H. Wagner, "Pharmazeutische Biologie - Drogen und ihre Inhaltsstoffe", Gustav Fischer Verlag, Stuttgart/New York, 1985, Seite 281, Stichwort: "Cetrariae Lichen (= Lichen islandicus - Isländisches Moos)".

Der Eibisch (*Althaea officinalis L*.), eingesetzt in Form der Eibischwurzel, -blätter und -blüten, wächst auf salzhaltigen und feuchten Böden Mittel-, Ost- und Südosteuropas. Die im Herbst geernteten, von der holzigen Hauptwurzel, von Wurzelfasern und Rindenschichten befreiten und bei 35 °C getrockneten Wurzelzweige und Nebenwurzeln kommen geschält oder ungeschält in den Handel. Die Braunfärbung der geschälte Droge bedeutet eine Qualitätsminderung; ein nachträgliches "Schönen" mit Sulfitlösung ist unzulässig. Weiterhin kommen die vor oder während der Blüte gesammelten, etwa 10 cm langen, etwa 8 cm breiten, drei- bis fünflappigen, graufilzig behaarten getrockneten Blätter und die im Juli/August gesammelten, fleischfarbenen getrockneten Kronblätter zur Anwendung. Der Eibisch ist eine mehrjährige, etwa 1 m hohe Staude, die generativ oder vegetativ vermehrt wird. Die im Spätherbst geerntete Wurzel enthält bis zu 15 % Schleimstoffe. Im Frühjahr und Sommer liegt der Schleimgehalt bei 5 bis 6 %. Der Schleimgehalt der Blatt- und Blütendroge beträgt 6 bis 9 %. Der Schleim ist in der Wurzel in bestimmten Schleimzellen des Rinden- und Holzparenchyms lokalisiert. Er besteht aus Galacturonorhamnanen, Glucanen und Arabinogalactanen. Wäßrige Auszüge aus der Wurzel sollen durch Mazeration bei Zimmertemperatur hergestellt werden, damit die reichlich vorhandene Stärke nicht durch Quellung störend wirkt. Eibischdrogen werden für Teezubereitungen oder zur Herstellung von Sirupus Althaeae verwendet. Das Hauptanwendungsgebiet sind entzündliche Reizzustände des Rachenraums. Äußerlich kommt der Eibisch zu erweichenden Umschlägen, Bädern und Kataplasmen zur Anwendung.

Die Malve (*Malva sylvestris L.* und *M. neglecta WALLR*.), insbesondere eingesetzt in Form von Malvenblüten und -blättern, ist in Europa, Kleinasien, im Mittelmeergebiet und in Vorderindien heimisch. Zur Anwendung kommen die zur Blütezeit gesammelten, rosa-violett gefärbten Blüten bzw. die durch das Trocknen stark eingeschrumpften, gefalteten Blätter, die zumeist in Paketform in den Handel kommen. Die Malve ist ein zwei- bis mehrjähriges Kraut. Der Schleimgehalt von Blüten und Blättern liegt bei 6 bis 8 %. Bei der Hydrolyse liefert der Schleim Glucose, Arabinose, Rhamnose und Galactose. Malvenblüten werden zu Gurgelwässern und Bädern und zusammen mit den Blättern in Teemischungen als Expectorans verwendet.

Der Bockshornklee (*Trigonella foenum-graecum L*.), insbesondere eingesetzt in Form des Bockshornkleesamens, ist im gesamten Mittelmeergebiet, sowie in Osteuropa, Indien und China beheimatet. Der Hauptdrogenimport stammt aus den Kulturen Indiens und Marokkos. Zur Anwendung kommen die braun-rötlich gefärbten, vierseitigen und rautenförmigen, etwa 5 mm langen und etwa 3 mm breiten, flachgedrückten, sehr harten Samen, die durch eine Furche charakterisiert sind. Die Droge enthält außer fettem Öl 20 bis 30 % Schleim, Trigonellin, Nicotinsäureamid, Cholin, Bitterstoffe und Saponine. Die Herstellung des Schleims erfolgt aus dem gepulvertem Samen. Das Pulver wird angewendet äußerlich zu Umschlägen bei Furunkeln, Geschwüren und Drüsenschwellungen, innerlich als Expectorans und Roborans.

Salep bzw. Salep Tuber, insbesondere eingesetzt in Form der Salepknolle, ist von verschiedenen Orchideen-Arten abgeleitet, besonders *Orchis mascula L., Orchis morio L., Orchis militaris L., Anacamptis pyramidalis L. RICH.* und *Platanthera bifolia L. RICH.* Zur Anwendung kommen die etwa 4 cm langen bis etwa 3 cm dicken, runzeligen, harten, bräunlichen Knollen, die nach der Ernte von der Korbschicht befreit, mit siedendem Wasser abgebrüht und bei künstlicher Wärme getrocknet werden. Salepschleim, der bis zu 50 % vorwiegend aus Glucomannan bzw. Glucan besteht, wird hauptsächlich in der Kinderheilpraxis als Antidiarrhoikum verwendet.

Die Quitte (*Cydonia oblonga MILL.*) ist im südöstlichen Arabien heimisch. Hautdrogenimporte kommen aus Spanien, Portugal und Persien. Zur Anwendung kommen die reifen, getrockneten, rot-braun bis braun-violett gefärbten, etwas abgeplatteten Samen der Quittenscheinfrucht. Die Samen sind außen zum Teil mit einer eingetrockneten Schleimkruste versehen und oft miteinander verklebt. Sie besitzen einen schwachen Bittermandelgeschmack. In der Epidermis findet sich ca. 22 % Schleim, der zum größten Teil wasserlöslich ist. Die Zuckerkomponenten sind Arabinose, Xylose und Uronsäure, die zum Teil methyliert ist. Die Droge kommt nur unzerkleinert zur Anwendung. Der Quittenschleim wird äußerlich bei Lippen- und Brustwarzenrhagaden, bei Verbrennungen und Dekubitus in Salben- oder Cremeform verwendet.

Erfindungsgemäß besonders gute Ergebnisse werden mit Spitzwegerich (*Plantago lanceolata L.)* erzielt.

Für weitere Einzelheiten zu Schleimdrogen und ihren Zubereitungen, Wirkungen und Anwendungen kann verwiesen werden auf H. Wagner "Pharmazeutische Biologie - Drogen und ihre Inhaltsstoffe", Gustav Fischer Verlag, Stuttgart/New York, 1985, insbesondere Seiten 280 ff.

Wie zuvor angeführt, sollte die Schleimdroge in Form eines Extraktes, insbesondere eines Trockenextraktes, zugesetzt sein. Ein solcher Trockenextrakt ist vorteilhafterweise auf Basis eines wäßrigen, alkoholischen oder wäßrig-alkoholischen Extraktes, bevorzugt auf Basis eines wäßrigen Extraktes, erhältlich (z. B. durch übliche Methoden, wie beispielsweise Abziehen des Wassers und/oder Alkohols im Vakuum, gegebenenfalls unter Erwärmen, Lyophilisation etc.). In vorteilhafter Weise kann im Rahmen der Erfindung ein Trockenextrakt von *Plantago lanceolata* eingesetzt werden, wobei als Drogenteile *Plantago lanceolata folia* eingesetzt werden. Als Auszugsmittel kann Ethanol (z. B. 40 Vol.-%) eingesetzt werden. Das Droge/Extraktzubereitung-Verhältnis kann hierbei z. B. etwa 4 : 1 und das Verhältnis von Droge zu Drogenzubereitung (nativer Extrakt) kann z. B. etwa 4,2 - 5,5 : 1 betragen. Der native Extraktanteil kann z. B. im Bereich von etwa 72 bis 95 Gew.-%, bezogen auf die Extraktzubereitung, liegen. Bei diesem Trockenextrakt handelt es sich um ein braungrünes feines Pulver mit schwachem Geruch und leicht bitterem Geschmack.

Die erfindungsgemäße Zusammensetzung sollte die Wirkstoffkomponenten (a) und/oder (b), insbesondere die Gerbstoffdroge und/oder die Schleimdroge, jeweils in einer Matrix und/oder Masse enthalten, insbesondere wobei die erste Wirkstoffkomponente (a), insbesondere die Gerbstoffdroge, in einer festen, insbesondere beim Lutschen und/oder unter Speicheleinwirkung auflösbaren Matrix und/oder Masse eingelagert ist und/oder insbesondere wobei die zweite Wirkstoffkomponente (b), insbesondere die Schleimdroge, in einer flüssigen, insbesondere beim Lutschen und/oder unter Speicheleinwirkung auflösbaren Matrix und/oder Masse eingelagert ist.

In diesem Zusammenhang sollte die erfindungsgemäße Zusammensetzung die Wirkstoffkomponenten (a) und/oder (b), insbesondere die Gerbstoffdroge und/oder die Schleimdroge, unabhängig voneinander, jeweils in einer Matrix und/oder Masse auf Basis von Zuckern und/oder Zuckeraustauschstoffen enthalten.

Was die erfindungsgemäße Zusammensetzung weiterhin anbelangt, so kann diese Zucker und/oder Zuckeraustauschstoffe in Mengen von 50 bis 99,6 Gew.-%, insbesondere 60 bis 99,5 Gew.-%, bevorzugt 70 bis 99 Gew.-%, besonders bevorzugt 80 bis 98,8 Gew.-%, ganz besonders bevorzugt 90 bis 98,5 Gew.-%, enthalten, bezogen auf die pharmazeutische Zusammensetzung.

Dabei sollten die Zucker ausgewählt sein aus der Gruppe von Saccharose, Glucose, insbesondere Dextrose, und Fructose. Die Zuckeraustauschstoffe sollten ausgewählt sein aus Zuckeralkoholen und/oder aus der Gruppe von Mannit, Xylit, Sorbit, Isomalt, insbesondere Flüssig-Isomalt, Maltitsirup, Lactit, Leucrose, Fructooligosacchariden, Glucanen und Polyglucose.

Besonders vorteilhaft ist es, wenn die die erste Wirkstoffkomponente (a) enthaltende erste Phase (A) und/oder die Hülle Isomalt, insbesondere Flüssig-Isomalt, enthält, insbesondere in Mengen von 70 bis 95 Gew.-%, insbesondere 75 bis 90 Gew.-%, bevorzugt 80 bis 90 Gew.-%, bezogen auf die Phase (A) und/oder die Hülle. Weiterhin kann die die erste Wirkstoffkomponente (a) enthaltende erste Phase (A) und/oder die Hülle Maltit, insbesondere Maltitsirup, enthalten, insbesondere in Mengen von 2 bis 20 Gew.-%, insbesondere 3 bis 18 Gew.-%, bevorzugt 5 bis 15 Gew.-%, bezogen auf die Phase (A) und/oder den Kern.

Weiterhin kann es erfindungsgemäß vorgesehen sein, daß die die zweite Wirkstoffkomponente (b) enthaltende zweite Phase (B) und/oder der Kern Maltit, insbesondere Maltitsirup, enthält, insbesondere in Mengen von 50 bis 95 Gew.-%, insbesondere 60 bis 90 Gew.-%, bevorzugt 70 bis 85 Gew.-%, bezogen auf die zweite Phase (B) und/oder den Kern.

In erfindungsgemäß bevorzugter Ausführungsform liegt die erfindungsgemäße Zusammensetzung in einer Darreichungsform auf Basis einer Lutschtablette vor, insbesondere in Form einer Hartkaramelle, einer Pastille oder Tablette. Die Darreichungsform kann auf Lutschtablettenbasis gebildet sein, insbesondere in Form einer mehrphasigen, vorzugsweise zweiphasigen Lutschtablettenbasis, vorzugsweise mit mindestens einer vorzugsweise festen Hülle und/oder festen ersten Phase (A), welche die erste Wirkstoffkomponente (a) enthält, und mit einem flüssigen Kern und/oder einer flüssigen zweiten Phase (B), welcher bzw. welche die zweite Wirkstoffkomponente (b) enthält.

In diesem Zusammenhang sollte die Darreichungsform der erfindungsgemäßen Zusammensetzung eine jeweils therapeutisch wirksame Menge der ersten Wirkstoffkomponente (a), insbesondere der Gerbstoffdroge, und der zweiten Wirkstoffkomponente (b), insbesondere der Schleimdroge, beim Lutschen und/oder unter Speicheleinfluß zeitversetzt freisetzen, wenn die Darreichungsform verabreicht und im Mundraum des Patienten gelutscht wird, wobei in zeitlicher Abfolge zuerst die erste Wirkstoffkomponente (a), insbesondere die Gerbstoffdroge, und nachfolgend die zweite Wirkstoffkomponente (b) freigesetzt wird.

In diesem Zusammenhang hat es sich, wie zuvor angeführt, als besonders vorteilhaft erwiesen, wenn die Zusammensetzung insbesondere in Form einer Lutschtablette mit einer festen Hülle und/oder einer festen ersten Phase (A) und mit einem flüssigen Kern und/oder einer flüssigen zweiten Phase (B) vorliegt.

Mit anderen Worten besteht das Grundprinzip der vorliegenden Erfindung somit darin, daß zunächst der *Pelargonium*-Bestandteil und darauffolgend bzw. zeitlich versetzt der Spitzwegerich-Bestandteil freigesetzt wird. d. h. die *Pelargonium-* und Spitzwegerich-Bestandteile werden zeitlich nacheinander freigesetzt, wobei die vorliegende Erfindung auch solche Ausführungsformen mitumfaßt, bei denen eine gewisse Überlappung der jeweiligen Freisetzung von *Pelargonium-*Bestandteilen einerseits und *Spitzwegerich*-Bestandteilen andererseits vorliegt. Wie zuvor angeführt, resultiert durch das versetzte Freisetzen eine besonders gute Wirksamkeit. In diesem Zusammenhang ist es aufgrund der festen Ausbildung der Hülle bzw. der ersten Phase (A) mit der Wirkstoffkomponente (a) gewährleistet, daß über einen zeitlich relativ langen Zeitraum geringe Mengen der Gerbstoffe freigesetzt werden, was hinsichtlich der Interaktion mit der (Mund-)Schleimhaut vorteilhaft ist. Durch die Freisetzung der zweiten Wirkstoffkomponente (b) aus der flüssigen Phase (B) bzw. dem flüssigen Kern wird dann eine rasche und effektive Bildung der Schleimschicht auf der zuvor mit den Gerbstoffen behandelten (Mund-)Schleimhaut erreicht, was zu einer weiter verbesserten Wirksamkeit der erfindungsgemäßen Zusammensetzung führt.

Wie zuvor beschrieben, eignet sich die erfindungsgemäße pharmazeutische Zubereitung insbesondere zur prophylaktischen und/oder therapeutischen Behandlung von entzündlichen Erkrankungen des Mund- und Rachenraums, Schleimhautreizungen und Schleimhautläsionen im Mund- und Rachenraum, Austrocknungen der Schleimhäute im Bereich des Mund- und Rachenraums, Husten und Hustenreiz, insbesondere trockenem Reizhusten, Heiserkeit, Bronchialkatharren und Bronchialasthma. In diesem Zusammenhang wird die erfindungsgemäße pharmazeutische Zubereitung topisch bzw. peroral appliziert.

Die Herstellung der erfindungsgemäßen pharmazeutischen Zubereitung kann in an sich bekannter Art und Weise erfolgen. Für weitergehende Einzelheiten kann auf die nachfolgenden Ausführungsbeispiele verwiesen werden.

In diesem Zusammenhang sollte die erfindungsgemäße Zusammensetzung insbesondere in Form einer Lutschtablette mit einem Gesamtgewicht von 1 bis 6 g, vorzugsweise 1,5 bis 5 g, besonders bevorzugt 2 bis 4 g, vorliegen.

Dabei sollte das gewichtsbezogene Verhältnis der festen Hülle und/oder der festen ersten Phase (A) einerseits zu dem flüssigen Kern und/oder der flüssigen zweiten Phase (B) andererseits im Bereich von 50 - 98 : 50 - 2, insbesondere 60 - 95 : 40 - 5, vorzugsweise 70 - 90 : 30 - 10, liegen.

Zudem sollte die Darreichungsform, insbesondere die Lutschtablette, 10 bis 500 mg, insbesondere 20 bis 400 mg, vorzugsweise 30 bis 300 mg, der ersten Wirkstoffkomponente (a), insbesondere der Gerbstoffdroge und/oder deren Extrakt, enthalten, insbesondere wobei die erste Wirkstoffkomponente (a), insbesondere Gerbstoffdroge und/oder deren Extrakt, zumindest im wesentlichen ausschließlich in der festen Hülle und/oder in der festen ersten Phase (A) enthalten ist. Bevorzugterweise sollte somit die erste Wirkstoffkomponente (a), insbesondere Gerbstoffdroge bzw. deren Extrakt, zumindest im wesentlichen ausschließlich in der festen Hülle und/oder in der festen ersten Phase (A) enthalten sein.

Zudem sollte die Darreichungsform, insbesondere die Lutschtablette, 5 bis 250 mg, insbesondere 10 bis 200 mg, vorzugsweise 15 bis 150 mg, der zweiten Wirkstoffkomponente (b), insbesondere der Schleimdroge und/oder deren Extrakt, enthalten. In diesem Zusammenhang sollte die zweite Wirkstoffkomponente (b), insbesondere Schleimdroge bzw. deren Extrakt, zumindest im wesentlichen ausschließlich in dem flüssigen Kern und/oder in der flüssigen zweiten Phase (B) enthalten sein.

Die Darreichungsform, insbesondere die Lutschtablette, kann zudem 1,5 bis 4,9 g, insbesondere 1,5 bis 2,9 g, Zucker und/oder Zuckeraustauschstoffe enthalten.

Die zuvor genannten Gewichtsangaben hinsichtlich der ersten Wirkstoffkomponente (a), welche die Gerbstoffdroge enthält bzw. darstellt, der zweiten Wirkstoffkomponente (b), welche die Schleimdroge enthält bzw. darstellt, sowie des Zuckers bzw. der Zuckeraustauschstoffe beziehen sich auf eine Dosiereinheit, insbesondere in Form einer Lutschtablette.

Zudem kann es erfindungsgemäß vorgesehen sein, daß die Zusammensetzung nach der Erfindung außerdem weitere Wirk- und/oder Inhaltsstoffe, insbesondere ausgewählt aus der Gruppe von Verarbeitungshilfsmitteln, Aromastoffen, Geschmacksstoffen, Süßstoffen und Süßungsmitteln, Säuerungsmitteln, Stabilisatoren und/oder Antiseptika, enthält.

Diese sind dem Fachmann als solche bekannt, und der Fachmann ist jederzeit in der Lage, die jeweiligen weiteren Wirk- bzw. Inhaltsstoffe hinsichtlich ihrer Art und Menge im Rahmen der erfindungsgemäßen Zusammensetzung auszuwählen und aufeinander abzustimmen.

Die erfindungsgemäße Zusammensetzung kann durch Lutschen, z. B. bei Erkältungskrankheiten eingesetzt werden, wodurch die Erkältungssymptome signifikant gelindert werden und zudem ein reflektorisch ausgelöster Hustenreiz vermindert wird. Durch den mechanischen Reiz des Lutschens wird zudem die Speichelbildung angeregt, was gleichermaßen von Vorteil ist. Die Gerbstoffe, welche insbesondere aus *Pelargonium*-Arten*,* wie *Pelargonium sidoides* und/oder *Pelargonium reniforme,* stammen, vernetzen bei der Anwendung die Schleimhautproteine und beschleunigen das Abheilen der bereits angegriffenen Schleimhaut. Die reizmildernden Schleimstoffe, insbesondere aus *Plantago lanceolata,* bilden einen Film auf der entzündeten Schleimhaut und schützen sie vor weiteren Angriffen durch trockene und gegebenenfalls schadstoffbelastete Atemluft und vermindern den reflektorisch ausgelösten Hustenreiz. Somit resultiert im Rahmen der vorliegenden Erfindung gewissermaßen ein dreifach physikalischer Schutz aus vermehrtem Speichelfluß sowie Gerb- und Schleimstoffen, was zu einer signifikanten Linderung und schnelleren Abheilung führt. Durch die erfindungsgemäß bevorzugte Ausführungsform mit der zeitversetzten Freisetzung der Gerbstoffdroge einerseits und der Schleimdroge andererseits wird die Wirksamkeit weiter erhöht. Wie zuvor angeführt, kann die hervorragende Wirksamkeit der erfindungsgemäßen Zusammensetzung noch weiter gesteigert werden, wenn die Gerbstoffdroge in einer festen Hülle und die Schleimstoffdroge in einem flüssigen Kern eingebracht sind.

Weiterer Gegenstand der vorliegenden Erfindung - gemäß einem weiterer Aspekt der vorliegenden Erfindung - ist eine erfindungsgemäße Verpackungseinheit, insbesondere Blisterverpackung, enthaltend die zuvor definierte Zusammensetzung, insbesondere in Form einer zur Einzeldosierung geeigneten Form, vorzugsweise in Form von Lutschtabletten. Die erfindungsgemäße Verpackungseinheit enthält vorzugsweise eine Mehrzahl an Lutschtabletten insbesondere zur Einzelentnahme.

Ein weiterer Gegenstand der vorliegenden Erfindung - gemäß einem wiederen weiteren Aspekt der vorliegenden Erfindung - ist die erfindungsgemäße Verwendung der Zusammensetzung nach der Erfindung, wie zuvor definiert, zur topischen Behandlung von entzündlichen Erkrankungen des Mund- und Rachenraums, Husten und Katharren der oberen Luftwegen bzw. die erfindungsgemäße Verwendung der Zusammensetzung nach der Erfindung, wie zuvor definiert, zur Herstellung eines Arzneimittels zur topischen Behandlung von entzündlichen Erkrankungen des Mund- und Rachenraums, Husten und Katharren der oberen Luftwege.

In diesem Zusammenhang sollte die erfindungsgemäße Zusammensetzung zur Verabreichung von 10 bis 5.000 mg, insbesondere 60 bis 3.000 mg der ersten Wirkstoffkomponente (a), insbesondere der Gerbstoffdroge und/oder deren Extrakt, und 5 bis 2.500 mg, insbesondere 30 bis 1.200 mg, der zweiten Wirkstoffkomponente (b), insbesondere der Schleimdroge und/oder deren Extrakt, pro Tag bei einer Verabreichung von 1 bis 12 Dosiereinheiten, insbesondere in Form einzelner Lutschtabletten, hergerichtet sein. Vorzugsweise sollten 3 bis 6 Dosiereinheiten pro Tag verabreicht werden, wobei die Verabreichung etwa alle 2 bis 3 Stunden erfolgen sollte.

Weitere Ausgestaltungen, Abwandlungen und Variationen sowie Vorteile der vorliegenden Erfindung sind für den Fachmann beim Lesen der Beschreibung ohne weiteres erkennbar und realisierbar, ohne daß er dabei den Rahmen der vorliegenden Erfindung verläßt.

Die folgenden Ausführungsbeispiele dienen lediglich der Veranschaulichung der vorliegenden Erfindung, ohne sich jedoch hierauf zu beschränken.

### Ausführungsbeispiele:

### Herstellungsbeispiel:

Eine pharmazeutische Zubereitung nach der vorliegenden Erfindung in Form einer Lutschtablette mit einer festen Hülle und einem flüssigen Kern wird ausgehend von den folgenden Zusammensetzungen für die feste Hülle und den flüssigen Kern hergestellt. Die nachfolgenden Prozentangaben beziehen sich auf die Gesamtzubereitung mit Hüll- und Kernzusammensetzung.

| 1. | Zusammensetzung für die feste Hülle: | |
|---|---|---|
| | Matrix-Bestandteile auf Basis von Zuckern und Zuckeralkoholen | 79,4442 Gew.-% |
| | Pelargonium-Urtinktur (1 : 8 - 9) | 3,3354 Gew.-% |
| | Bearbeitungshilfsmittel, Aromastoffe, Geschmacksstoffe, Säuerungsmittel | 0,5204 Gew.-% |
| | gesamt ca. | 83,3000 Gew.-% |
| | Restfeuchte (2 %) | 1,7000 Gew.-% |
| | Hülle | 85,0000 Gew.-% |

| 2. | Zusammensetzung für den flüssigen Kern: | |
|---|---|---|
| | Matrix-Bestandteile auf Basis von Zuckern und Zuckeralkoholen | 11,8731 Gew.-% |
| | Spitzwegerich-Pulverextrakt (4 : 1) | 0,8343 Gew.-% |
| | Bearbeitungshilfsmittel, Aromastoffe, Geschmacksstoffe, Säuerungsmittel | 0,0425 Gew.-% |
| | gesamt ca. | 12,7499 Gew.-% |
| | Restfeuchte (15 %) | 2,2500 Gew.-% |
| | Hülle | 15,0000 Gew.-% |

Die jeweiligen Zusammensetzungen für die Hülle und für den Kern werden zu erfindungsgemäßen Lutschtabletten mit festem Mantel bzw. mit fester Hülle und mit flüssigem Kern bzw. flüssiger Füllung verarbeitet, wobei in bezug auf die Herstellung der Hülle zunächst aus den Matrix-Bestandteilen eine Grundmischung hergestellt wird und nach Vorlösen und Kochen ein Vakuumieren erfolgt. Anschließend werden die weiteren Komponenten, darunter die *Pelargonium-*Urtinktur, zudosiert. Die Masse wird auf ein Kühlband gepumpt und temperiert. Ein Kegelroller wird mit der so hergestellten Masse beschickt. Was die Füllung bzw. den flüssigen Kern anbelangt, so wird aus den hierfür eingesetzten Materialien zunächst eine Füllung gekocht und der erforderliche Restfeuchtegehalt eingestellt. Die Füllung bzw. der Kern wird mittels eines Füllrohrs in den Zuckerkegel des Kegelrollers kontinuierlich eindosiert, anschließend werden die erfindungsgemäßen Lutschtabletten auf einer speziellen Auszieh- und Prägemaschine auf die vorgegebene Dimensionierung ausgeformt. Das Gewicht der erfindungsgemäßen Lutschtablette wird dabei auf etwa 3 g eingestellt. Das Hülle/Kern-Verhältnis beträgt etwa 85 : 15. Die erfindungsgemäßen Lutschtabletten gelangen über eine Kühlanlage und einer anschließenden Sortier- und Kontrollstrecke in Bulkkartons. Nachfolgend können entsprechende Blisterverpackungen befüllt werden.

### Anwendungsbeobachtungen bzw. Wirksamkeitsstudien:

1.) 15 Patienten im Alter von 28 bis 45 Jahren mit einer Irritation der Schleimhäute im Mund- und Rachenbereich infolge von Erkältungserkrankungen wurden mit der im vorangehenden Herstellungsbeispiel beschriebenen pharmazeutischen Zubereitung auf Basis von Lutschtabletten mit fester Hülle und flüssigem Kern therapiert, wobei über einen Zeitraum von zehn Tagen jeweils sechs Tabletten am Tag verabreicht wurden (Gruppe A).
   Weitere 15 Patienten im Alter von 31 bis 42 Jahren mit einer zu der Gruppe (A) vergleichbaren Beschwerdesymptomatik wurden mit Lutschtabletten behandelt, welche zu dem Herstellungsbeispiel vergleichbare Gehalte an Tinkturen aufwiesen. Die Lutschtabletten wiesen eine feste Hülle und einen festen Kern auf. Die Zusammensetzung wurde über einen Zeitraum von zehn Tagen mit täglich jeweils sechs Lutschtabletten appliziert (Gruppe B).
   Einer weiteren Patientengruppe, welche aus zehn Patienten im Alter von 31 bis 45 Jahren mit der zuvor beschriebenen Beschwerdesymptomatik umfaßte, wurde ein Monopräparat auf Basis einer festen Lutschtablette verabreicht, welche lediglich eine Gerbstoffdroge auf Basis von *Pelargonium* enthielt. Der Gehalt an *Pelargonium*-Urtinktur entsprach dem in dem Herstellungsbeispiel beschriebenen Gehalt (Gruppe C).
   Im Rahmen einer weiteren Untersuchungsgruppe aus zehn Patienten im Alter von 31 bis 46 Jahren wurden Lutschtabletten nach Art eines Monopräparates verabreicht, wobei die Tabletten in fester Form vorlagen und nur eine Plantago-Schleimdroge enthielten. Der Gehalt an *Plantago-*Pulverextrakt entsprach dem in dem Herstellungsbeispiel beschriebenen Gehalt. Über einen Zeitraum von zehn Tagen wurden sechs Lutschtabletten pro Patient und pro Tag verabreicht (Gruppe D).
   Während bei sämtlichen Patienten der Gruppe A die Beschwerden bereits nach fünf Tagen vollständig abgeklungen waren, d. h. die entzündlichen Symptome an den Mund- und Rachenschleimhäuten nicht mehr beobachtet werden konnten, wurde in bezug auf die Patienten der Gruppe B bei acht Patienten ein Abklingen der Symptome nach sechs Tagen beobachtet werden, während bei zwei Patienten der Gruppe B die Symptome nach sieben Tagen abgeklungen waren. Sowohl die Probanden der Gruppe A als auch die Probanden der Gruppe B berichteten bereits am ersten Tag der Verabreichung der jeweiligen Zusammensetzung eine deutliche Linderung der Beschwerdesymptome. Zudem beurteilten sämtliche Probanden der Gruppe A das Lutschgefühl der erfindungsgemäßen Lutschtablette als sehr angenehm, insbesondere wurde das "Zerfließen" des flüssigen Kernes als wohltuend empfunden.
   Bei den Probanden der Gruppe C konnte bei acht Probanden eine deutliche Linderung bzw. ein Ausheilen der Beschwerdesymptome nach neun Tagen beobachtet werden, bei zwei Probanden war auch nach zehn Tagen noch kein vollständiges Abklingen der Beschwerden eingetreten. Was die Probanden der Gruppe D anbelangt, so wiesen nach zehn Tagen noch drei Patienten deutliche Symptome hinsichtlich einer entzündlichen Schleimhautirritation auf, bei fünf Patienten waren nur noch schwache Symptome zu beobachten, während zwei Patienten nach zehn Tagen nahezu beschwerdefrei waren.
   Die Untersuchungen zeigen, daß die Anwendung der erfindungsgemäßen Zubereitung in Form einer Kombination von Gerbstoffen und Schleimdroge mit der zeitversetzten Freisetzung der jeweiligen Komponenten und dem flüssigen Kern einen deutlich gesteigerten Therapieerfolg wirkt, da durch die erfindungsgemäße Zubereitung ein verbesserter Therapieerfolg - Verschwinden der Symptome - erreicht wird. Zudem wurde die Anwendung der erfindungsgemäßen Zusammensetzung durch die Probanden als angenehm empfunden.
   Die erfindungsgemäße pharmazeutische Zubereitung führt bei der Behandlung von Irritationen der Schleimhäute des Mund- und Rachenraumes zu einer eindrucksvollen Besserung der hiermit einhergehenden Symptome. Hierin liegt ein entscheidender Vorteil der erfindungsgemäßen Kombination.
2.) Im Rahmen einer Untersuchungsreihe wurde die antivirale Wirkung der erfindungsgemäßen Zusammensetzung, wie sie im Herstellungsbeispiel beschrieben ist, mit derjenigen des Monopräparates, wie es in bezug auf Gruppe D eingesetzt wurde, verglichen. Diesbezüglich wurden von den Probanden der Gruppe A und der Gruppen C und D in täglich Speichelproben entnommen und der diesbezügliche Virustiter von erkältungstypischen Viren bestimmt. Bei den Probanden der Gruppe A wurden im Vergleich zu den Probanden der Gruppen C und D signifikant verringerte Virustiter festgestellt werden, was die antivirale Wirksamkeit der erfindungsgemäßen Zusammensetzung belegt.

## Patentansprüche

1. Pharmazeutische Zusammensetzung in einer zum Lutschen geeigneten Darreichungsform zur topischen Behandlung von entzündlichen Erkrankungen des Mund- und Rachenraums, Husten und Katarrhen der oberen Luftwege, wobei die Zusammensetzung in pharmazeutisch wirksamen Mengen eine Kombination von
(a) 0,05 bis 30 Gew.-%, bezogen auf die pharmazeutische Zusammensetzung, mindestens einer ersten Wirkstoffkomponente, welche mindestens eine Gerbstoffdroge in Form eines Extraktes mit einem Droge/Extrakt-Verhältnis im Bereich von 1 : 0,1 bis 1 : 100 enthält, wobei die Gerbstoffdroge ausgewählt ist aus *Pelargonium,* Brombeere (*Rubus fructiosus*)*,* Eiche *(Quercus rubor* und/oder *Quercus petraea*), Gänsefingerkraut *(Potentilla anserina),* Gewürznelken (*Szygium aromaticum*)*,* Heidelbeere *(Vaccinium myrtillus),* Myrrhe (*Commiphora molmol*)*,* Ratanhia (*Krameria triandra*)*,* Salbei *(Salvia triloba),* Schlehdorn (*Prunus spinosa*), Taubnessel (*Lamium album*)*,* Blutwurz (*Potentilla erecta*)*,* Erdbeere (*Fragaria vesca*), Odermenningkraut (*Agrimonia eupatoria*)*,* Frauenmantelkraut (*Alchemilla xanthochlora*), Rose (*Rosa gallica),* Wiesenknopf *(Sanguisorba officinalis)* und deren Kombinationen; mit
(b) 0,01 bis 20 Gew.-%, bezogen auf die pharmazeutische Zusammensetzung, mindestens einer zweiten Wirkstoffkomponente, welche mindestens eine Schleimdroge in Form eines Extraktes mit einem Droge/Extrakt-Verhältnis im Bereich von 0,4 : 1 bis 15 : 1 enthält, wobei die Schleimdroge ausgewählt ist aus Spitzwegerich (*Plantago lanceolata L.),* Isländischem Moos (*Lichen islandicus*), Eibisch (*Althaea officinalis L*.)*,* Malve (*Malva sylvestris L.* und *M. neglecta WALLR.*), Boxhornklee (*Trigonella foenum-graecum L.*), Salep und Quitte *(Cydonia oblonga MILL.*) und deren Kombinationen;
umfasst, wobei die Zusammensetzung mindestens eine erste Phase (A) und mindestens eine von der ersten Phase (A) umgebene zweite Phase (B) aufweist, wobei die erste Phase (A) mindestens die erste Wirkstoffkomponente (a) aufweist und die zweite Phase (B) mindestens die zweite Wirkstoffkomponente (b) aufweist, wobei die erste Phase (A) als Hülle und die zweite Phase (B) als Kern ausgebildet ist, wobei die erste Phase (A) in fester, beim Lutschen und/oder unter Speicheleinfluss auflösbarer Form vorliegt und wobei die zweite Phase (B) in flüssiger Form vorliegt, wobei die Phasen (A) und (B) die Wirkstoffkomponenten (a) und (b) beim Lutschen und/oder unter Speicheleinfluss zeitversetzt freisetzen, wobei die erste Wirkstoffkomponente (a) beim Lutschen und/oder unter Speicheleinfluss zeitlich vor der zweiten Wirkstoffkomponente (b) freigesetzt wird, wobei die Zusammensetzung die Wirkstoffkomponenten (a) und/oder (b) unabhängig voneinander jeweils in einer Matrix und/oder Masse auf Basis von Zuckern und/oder Zuckeraustauschstoffen enthält und wobei das gewichtsbezogene Verhältnis der festen ersten Phase (A) einerseits zu der flüssigen zweiten Phase (B) andererseits im Bereich von 50 - 98 : 50 - 2 liegt.

2. Zusammensetzung nach Anspruch 1, wobei die zweite Phase (B) in einer dickflüssigen, insbesondere sirupösen, bis hin zu einer pastösen oder gar gelartigen Form ausgebildet ist, insbesondere wobei die dynamische Viskosität bei Raumtemperatur (20° C) und Umgebungsdruck (Atmosphärendruck) der zweiten Wirkstoffkomponente (b) und/oder der zweiten Phase (B) und/oder des Kerns im Bereich von 1 bis 1.000 mPa·s, insbesondere 5 bis 800 mPa·s, vorzugsweise 10 bis 500 mPa·s, bevorzugt 15 bis 300 mPa·s, besonders bevorzugt 20 bis 200 mPa·s, liegt.

3. Zusammensetzung nach Anspruch 1 oder 2, wobei die erste Wirkstoffkomponente (a) in Form einer Urtinktur zugesetzt ist und/oder wobei der Extrakt ausgehend von einem wässrigen, alkoholischen oder wässrig-alkoholischen Extrakt, bevorzugt einem wässrigalkoholischen Extrakt, erhältlich ist und/oder wobei die erste Wirkstoffkomponente (a), insbesondere die Gerbstoffdroge, in Form eines Extraktes, insbesondere Flüssigextraktes, vorzugsweise in Form einer Urtinktur, mit einem Droge/Extrakt-Verhältnis im Bereich von 1 : 0,5 bis 1 : 50, vorzugsweise 1 : 1 bis 1 : 20, bevorzugt 1 : 5 bis 1 : 15, zugesetzt ist.

4. Zusammensetzung nach einem der vorangehenden Ansprüche, enthaltend die erste Wirkstoffkomponente (a) in Mengen von 0,1 bis 20 Gew.-%, vorzugsweise 0,2 bis 10 Gew.-%, bevorzugt 0,5 bis 5 Gew.-%, bezogen auf die pharmazeutische Zusammensetzung.

5. Zusammensetzung nach einem der vorangehenden Ansprüche, wobei die Gerbstoffdroge *Pelargonium,* insbesondere *Pelargonium sidoides* und/oder *Pelargonium reniforme,* ist.

6. Zusammensetzung nach einem der vorangehenden Ansprüche, wobei die zweite Wirkstoffkomponente (b) in Form eines Trockenextraktes zugesetzt ist und/oder wobei der Extrakt ausgehend von einem wässrigen, alkoholischen oder wässrig-alkoholischen Extrakt, bevorzugt einem wässrigen Extrakt, erhältlich ist und/oder wobei die zweite Wirkstoffkomponente (b), insbesondere die Schleimdroge, in Form eines Extraktes, vorzugsweise eines Trockenextraktes, mit einem Droge/Extrakt-Verhältnis im Bereich von 1 : 1 bis 10 : 1, bevorzugt im Bereich von 5 : 1 bis 8 : 1, zugesetzt ist.

7. Zusammensetzung nach einem der vorangehenden Ansprüche, enthaltend die zweite Wirkstoffkomponente (b) in Mengen von 0,05 bis 10 Gew.-%, vorzugsweise 0,05 bis 5 Gew.-%, bevorzugt 0,08 bis 2 Gew.-%, bezogen auf die pharmazeutische Zusammensetzung.

8. Zusammensetzung nach einem der vorangehenden Ansprüche, wobei die Schleimdroge Spitzwegerich *(Plantago lanceolata L.)* ist.

9. Zusammensetzung nach einem der vorangehenden Ansprüche, enthaltend die Wirkstoffkomponenten (a) und/oder (b) unabhängig voneinander jeweils in einer Matrix und/oder Masse, wobei die erste Wirkstoffkomponente (a) in einer festen, beim Lutschen und/oder unter Speicheleinwirkung auflösbaren Matrix und/oder Masse eingelagert ist und/oder wobei die zweite Wirkstoffkomponente (b) in einer flüssigen, beim Lutschen und/oder unter Speicheleinwirkung auflösbaren Matrix und/oder Masse eingelagert ist.

10. Zusammensetzung nach einem der vorangehenden Ansprüche, wobei die Zusammensetzung Zucker und/oder Zuckeraustauschstoffe in Mengen von 50 bis 99,6 Gew.-%, insbesondere 60 bis 99,5 Gew.-%, bevorzugt 70 bis 99 Gew.-%, besonders bevorzugt 80 bis 98,8 Gew.-%, ganz besonders bevorzugt 90 bis 98,5 Gew.-%, bezogen auf die pharmazeutische Zusammensetzung, enthält.

11. Zusammensetzung nach einem der vorangehenden Ansprüche, wobei die die erste Wirkstoffkomponente (a) enthaltende erste Phase (A) Isomalt, insbesondere Flüssig-Isomalt, enthält, insbesondere in Mengen von 70 bis 95 Gew.-%, insbesondere 75 bis 90 Gew.-%, bevorzugt 80 bis 90 Gew.-%, bezogen auf die Phase (A), und/oder wobei die die erste Wirkstoffkomponente (a) enthaltende erste Phase (A) Maltit, insbesondere Maltitsirup, enthält, insbesondere in Mengen von 2 bis 20 Gew.-%, insbesondere 3 bis 18 Gew.-%, bevorzugt 5 bis 15 Gew.-%, bezogen auf die Phase (A), und/oder wobei die die zweite Wirkstoffkomponente (b) enthaltende zweite Phase (B) Maltit, insbesondere Maltitsirup, enthält, insbesondere in Mengen von 50 bis 95 Gew.-%, insbesondere 60 bis 90 Gew.-%, bevorzugt 70 bis 85 Gew.-%, bezogen auf die zweite Phase (B).

12. Zusammensetzung nach einem der vorangehenden Ansprüche, wobei die Darreichungsform eine Lutschtablette ist, insbesondere in Form einer Hartkaramelle, einer Pastille oder Tablette, und/oder wobei die Darreichungsform auf Lutschtablettenbasis gebildet ist, insbesondere in Form einer mehrphasigen, vorzugsweise zweiphasigen Lutschtablettenbasis.

13. Zusammensetzung nach einem der vorangehenden Ansprüche, wobei das gewichtsbezogene Verhältnis der festen ersten Phase (A) einerseits zu der flüssigen zweiten Phase (B) andererseits im Bereich von 60 - 95 : 40 - 5, vorzugsweise 70 - 90 : 30 - 10, liegt.

14. Verpackungseinheit, insbesondere Blisterverpackung, enthaltend die Zusammensetzung nach einem der vorangehenden Ansprüche, insbesondere in Form einer zur Einzeldosierung geeigneten Form, vorzugsweise in Form von Lutschtabletten, insbesondere wobei die Verpackungseinheit eine Mehrzahl an Lutschtabletten insbesondere zur Einzelentnahme enthält.

15. Verwendung einer Zusammensetzung, wie in einem der vorangehenden Ansprüche definiert, zur Herstellung eines Arzneimittels zur topischen Behandlung von entzündlichen Erkrankungen des Mund- und Rachenraums, Husten und Katarrhen der oberen Luftwege.

## Claims

1. A pharmaceutical composition in a dosage form as a lozenge, for the topical treatment of inflammatory diseases of the mouth and throat area, cough and catarrhs of the upper respiratory system, wherein the composition comprises a combination of
(a) 0.05 to 30% by weight, based on the pharmaceutical composition, of at least one first active ingredient, containing at least one tanning agent in the form of an extract, having a drug/extract ratio in the range of 1:0.1 to 1:100, wherein the tanning agent is selected from *Geranium,* blackberry (*Rubus fructiosus*), oak *(Quercus rubor* and/or *Quercus petraea*)*,* silverweed *(Potentilla anserina),* clove (*Szygium aromaticum*), blueberry *(Vaccinium myrtillus*)*,* myrrh *(Commiphora molmol*)*,* Ratanhia (*Krameria triandra*)*,* sage *(Salvia triloba),* blackthorn (*Prunus spinosa*)*,* dead-nettle (*Lamium album*)*,* common tormentil (*Potentilla erecta*)*,* strawberry (*Fragarai vesca*)*,* agrimony (*Agrimonia eupatoria*)*,* lady's mantle (*Alchemilla xanthochlora*)*,* rose (*Rosa gallica*)*,* sanguisorba *(Sanguisorba officinalis),* and the combinations thereof; with
(b) 0.01 to 20% by weight, based on the pharmaceutical composition, of at least a second active ingredient, containing at least one mucilage drug in the form of an extract, having a drug/extract ratio in the range of 0.4:1 to 15:1, wherein the mucilage drug is selected from buckhorn plantain *(Plantago lanceolata L.),* Icelandic moss (*Lichen islandicus*)*,* lady's finger (*Althaea officinalis L*.)*,* hollyhock *(Malva sylvestris L.* and M. *neglecta WALLR*.)*,* fenugreek (*Trigonella foenum-graecum L.*), salep and quince (*Cydonia oblonga MILL.*), and the combinations thereof;
in pharmaceutically effective amounts, wherein the composition has at least a first phase (A), and at least a second phase (B) being surrounded by the first phase (A), wherein the first phase (A) has at least the first active ingredient component (a), and the second phase (B) has at least the second active ingredient component (b), wherein the first phase (A) is embodied as a sheath, and the second phase (B) as a core, wherein the first phase (A) is present in a solid form, which is dissolvable during sucking, and/or under the influence of saliva, and wherein the second phase (B) is present in a liquid form, wherein phases (A) and (B) release the active ingredient components (a) and (b) during sucking and/or under the influence of saliva, in a time-delayed manner, wherein the first active ingredient component (a) is chronologically released before the second active ingredient component (b) during sucking and/or under the influence of saliva, wherein the composition contains the active ingredient components (a) and/or (b) independently of each other, each in a matrix, and/or a mass based on sugars and/or sugar substitutes, and wherein the ratio that is based on both the weight of the solid first phase (A) and the liquid second phase (B) is in the range of 50-98:50-2.

2. The composition according to claim 1, wherein the second phase (B) is embodied in a viscous, in particular syrup-like, up to a paste-like, or even gel-like form, in particular, wherein the dynamic viscosity at room temperature (20°C), and an ambient pressure (atmospheric pressure) of the second active ingredient component (b), and/or of the second phase (B), and/or of the core is in the range of 1 to 1,000 mPa·s, in particular 5 to 800 mPa·s, preferably 10 to 500 mPa·s, more preferred 15 to 300 mPa·s, particularly preferred 20 to 200 mPa·s.

3. The composition according to claims 1 or 2, wherein the first active ingredient component (a) is added in the form of an elementary tincture, and/or wherein the extract is available based on an aqueous, alcoholic, or aqueous-alcoholic extract, preferably an aqueous-alcoholic extract, and/or wherein the first active ingredient component (a), in particular the tanning agent, is added in the form of an extract, in particular a liquid extract, preferably in the form of an elementary tincture, having a drug/extract ratio in the range of 1:0.5 to 1:50, preferably 1:1 to 1:20, more preferred 1:5 to 1:15.

4. The composition according to one of the previous claims, containing the first active ingredient component (a) at amounts from 0.1 to 20% by weight, preferably 0.2 to 10% by weight, more preferred 0.5 to 5% by weight, based on the pharmaceutical composition.

5. The composition according to one of the previous claims, wherein the tanning agent is *Pelargonium,* in particular *Pelargonium sidoides,* and/or *Pelargonium reniforme.*

6. The composition according to one of the previous claims, wherein the second active ingredient component (b) is added in the form of a dry extract, and/or wherein the extract is available based on an aqueous, alcoholic, or aqueous-alcoholic extract, preferably an aqueous-alcoholic extract, and/or wherein the second active ingredient component (b), in particular the mucilage drug, is added in the form of an extract, in particular a dry extract, preferably in the form of an elementary tincture, having a drug/extract ratio in the range of 1:1 to 10:1, preferably in the range of 5:1 to 8:1

7. The composition according to one of the previous claims, comprising the second active ingredient component (b) in amounts of 0.05 to 10% by weight, preferably 0.05 to 5% by weight, more preferred 0.08 to 2% by weight, based on the pharmaceutical composition.

8. The composition according to one of the previous claims, wherein the mucilage drug is buckhorn plantain *(Plantago lanceolata L.).*

9. The composition according to one of the previous claims, comprising the active ingredient component (a) and/or (b) independently of each other, each in a matrix and/or mass, wherein the first active ingredient component (a) is embedded in a solid matrix and/or mass that is dissolvable by means of sucking and/or under the influence of saliva, and/or wherein the second active ingredient component (b) is embedded in a matrix and/or mass that is dissolvable by means of sucking and/or under the influence of saliva.

10. The composition according to one of the previous claims, wherein the composition comprises sugar and/or sugar substitutes at amounts of 50 to 99.6% by weight, in particular 60 to 99.5%by weight, preferably 70 to 99% by weight, particularly preferred 80 to 98.8% by weight, most particularly preferred 90 to 98.5% by weight, based on the pharmaceutical composition.

11. The composition according to one of the previous claims, wherein the first phase (A) containing the first active ingredient component (a) comprises isomalt, in particular liquid isomalt, in particular at amounts of 70 to 95% by weight, in particular 75 to 90% by weight, preferably 80 to 90% by weight, based on phase (A), and/or wherein the first phase (A) containing the first active ingredient component (a) comprises maltite, in particular maltite syrup, in particular at amounts of 2 to 20% by weight, in particular 3 to 18% by weight, preferably 5 to 15% by weight, based on phase (A), and/or wherein the second phase (B) containing the second active ingredient component (b) comprises maltite, in particular maltite syrup, in particular at amounts of 50 to 95% by weight, in particular 60 to 90% by weight, preferably 70 to 85% by weight, based on the second phase (B).

12. The composition according to one of the previous claims, wherein the dosage form is a lozenge, in particular in the form of hard candy, a pastille, or tablet, and/or wherein the dosage form is based on a lozenge, in particular in the form of a multiphase, preferably dual phase lozenge base.

13. The composition according to one of the previous claims, wherein the ratio based on the weight of the solid first phase (A) and the liquid second phase (B) is in the range of 60-95:40-5, preferably 70-90:30-10.

14. A packaging unit, in particular blister package, comprising the composition according to one of the previous claims, in particular in the form of a form suitable for single doses, preferably in the form of lozenges, in particular, wherein the packaging unit comprises a plurality of lozenges, in particular for individual removal.

15. A use of a composition as defined in one of the previous claims, for the production of a pharmaceutical for the topical treatment of inflammatory diseases of the mouth and throat area, cough, and catarrhs of the upper respiratory system.

## Revendications

1. Composition pharmaceutique sous une forme galénique convenant au suçage, pour le traitement topique de maladies inflammatoires de la cavité buccale et du pharynx, de la toux et des catarrhes des voies respiratoires supérieures, la composition comprenant des quantités à effet pharmaceutique d'une combinaison
(a) de 0,05 à 30 % en poids, par rapport à la composition pharmaceutique, d'au moins un premier composant principe actif, qui contient au moins une drogue astringente sous forme d'un extrait présentant un rapport drogue/extrait compris dans la plage de 1:0,1 à 1:100, la drogue astringente étant choisie parmi *Pelargonium*, la mûre sauvage (*Rubus fructiosus*), le chêne (*Quercus rubor* et/ou *Quercus petraea*)*,* la potentille ansérine *(Potentilla anserina*)*,* le giroflier (*Szygium aromaticum*)*,* les myrtilles *(Vaccinium myrtillus),* la myrrhe *(Commiphora molmol),* le ratanhia (*Krameria triandra*)*,* la sauge *(Salvia triloba),* le prunellier sauvage (*Prunus spinosa*)*,* l'ortie blanche (*Lamium album*), la tormentille (*Potentilla erecta*)*,* le fraisier (*Fragaria vesca*), l'agrimoine eupatoire (*Agrimonia eupatoria*)*,* l'alchemilles vulgaire (*Alchemilla xanthochlora*)*,* la rose (*Rosa gallica*)*,* la sanguisorbe officinale (*Sanguisorba officinalis)* et leurs combinaisons ; avec
(b) de 0,01 à 20 % en poids, par rapport à la composition pharmaceutique, d'au moins un deuxième composant principe actif, qui contient au moins une drogue mucilagineuse sous forme d'un extrait ayant un rapport drogue/extrait compris dans la plage de 0,4:1 à 15:1, la drogue mucilagineuse étant choisie parmi le plantain lancéolé *(Plantago lanceolata L.),* la mousse d'Islande (*Lichen islandicus*), la guimauve officinale (*Althaea officinalis L*.), la mauve (*Malva sylves tris L.* et *M*. *neglecta WALLR*.), la trigonelle fénugrec (*Trigonella foenum-graecum L.*), le salep et le coing (*Cydonia oblonga MILL*.) et leurs combinaisons ;
la composition présentant au moins une première phase (A) et au moins une deuxième phase (B) entourée par la première phase (A), la première phase (A) comprenant au moins un premier composant principe actif (a) et la deuxième phase (B) comprenant au moins un deuxième composant principe actif (b), la première phase (A) étant configurée comme une enveloppe et la deuxième phase (B) comme un noyau, la première phase (A) se présentant sous forme solide pouvant se dissoudre lors du suçage et/ou sous l'action de la salive, et la deuxième phase (B) se présentant sous forme liquide, les phases (A) et (B) libérant avec un décalage dans le temps les composants principes actifs (a) et (b) lors du suçage et/ou sous l'influence de la salive, le premier composant principe actif (a) étant, lors du suçage et/ou sous l'action de la salive, libéré dans le temps avant le deuxième composant principe actif (b), la composition contenant les composants principes actifs (a) et/ou (b), chacun indépendamment l'un de l'autre, dans une matrice et/ou une masse à base de sucres et/ou de succédanés du sucre, et le rapport en poids entre la première phase solide (A) d'une part et la deuxième phase liquide (B) d'autre part étant compris dans la plage de 50-98:50-2.

2. Composition selon la revendication 1, dans laquelle la deuxième phase (B) est configurée sous une forme épaisse, en particulier sirupeuse, jusqu'à une forme pâteuse, ou même de type gel, en particulier dans laquelle la viscosité dynamique à la température ambiante (20°C) et sous la pression ambiante (pression atmosphérique) du deuxième composant principe actif (b) et/ou de la deuxième phase (B) et/ou du noyau est comprise dans la plage de 1 à 1000 mPa·s, en particulier de 5 à 800 mPa·s, de préférence de 10 à 500 mPa·s, d'une manière préférée de 15 à 300 mPa·s, d'une manière particulièrement préférée de 20 à 200 mPa·s.

3. Composition selon la revendication 1 ou 2, dans laquelle le premier composant principe actif (a) est ajouté sous forme d'une teinture-mère, et/ou dans laquelle l'extrait peut être obtenu à partir d'un extrait aqueux, alcoolique ou hydro-alcoolique, de préférence d'un extrait hydro-alcoolique, et/ou dans laquelle le premier composant principe actif (a), en particulier la drogue astringente, est ajouté sous forme d'un extrait, en particulier d'un extrait liquide, de préférence sous forme d'une teinture-mère, ayant un rapport drogue/extrait compris dans la plage de 1:0,5 à 1:50, de préférence de 1:1 à 1:20, d'une manière particulièrement préférée de 1:5 à 1:15.

4. Composition selon l'une des revendications précédentes, contenant le premier composant principe actif (a) en des quantités de 0,1 à 20 % en poids, de préférence de 0,2 à 10 % en poids, d'une manière préférée de 0, 5 à 5 % en poids par rapport à la composition pharmaceutique.

5. Composition selon l'une des revendications précédentes, dans laquelle la drogue astringente est *Pelargonium,* en particulier *Pelargonium sidoides* et/ou *Pelargonium reniforme.*

6. Composition selon l'une des revendications précédentes, dans laquelle le deuxième composant principe actif (b) est ajouté sous forme d'un extrait sec et/ou dans laquelle l'extrait peut être obtenu à partir d'un extrait aqueux, alcoolique ou hydro-alcoolique, de préférence d'un extrait aqueux, et/ou dans laquelle le deuxième composant principe actif (b), en particulier la drogue mucilagineuse, est ajouté sous forme d'un extrait, de préférence d'un extrait sec, ayant un rapport drogue/extrait compris dans la plage de 1:1 à 10:1, de préférence dans la plage de 5:1 à 8:1.

7. Composition selon l'une des revendications précédentes, contenant le deuxième composant principe actif (b) en des quantités de 0,05 à 10 % en poids, de préférence de 0,05 à 5 % en poids, d'une manière préférée de 0,08 à 2 % en poids, par rapport à la composition pharmaceutique.

8. Composition selon l'une des revendications précédentes, dans laquelle la drogue mucilagineuse est le plantain lancéolé *(Plantago lanceolata L.).*

9. Composition selon l'une des revendications précédentes, contenant les composants principes actifs (a) et/ou (b), chacun indépendamment l'un de l'autre dans une matrice et/ou une masse, le premier composant principe actif (a) étant incorporé dans une matrice et/ou dans une masse solide, pouvant se dissoudre lors du suçage et/ou sous l'action de la salive, et/ou le deuxième composant principe actif (b) étant incorporé dans une matrice et/ou une masse liquide pouvant se dissoudre lors du suçage et/ou sous l'action de la salive.

10. Composition selon l'une des revendications précédentes, dans laquelle la composition contient des sucres et/ou des succédanés du sucre en des quantités de 50 à 99, 6 % en poids, en particulier de 60 à 99,5 % en poids, d'une manière préférée de 70 à 99 % en poids, d'une manière particulièrement préférée de 80 à 98,8 % en poids, d'une manière tout particulièrement préférée de 90 à 98,5 % en poids, par rapport à la composition pharmaceutique.

11. Composition selon l'une des revendications précédentes, dans laquelle la première phase (A) contenant le premier composant principe actif (a) contient de l'isomalt, en particulier en des quantités de 70 à 95 % en poids, en particulier de 75 à 90 % en poids, d'une manière préférée 80 à 90 % en poids, par rapport à la phase (A), et dans laquelle la première phase (A) contenant le premier composant principe actif (a) contient du maltitol, en particulier du sirop de maltitol, en particulier en des quantités de 2 à 20 % en poids, en particulier de 3 à 18 % en poids, d'une manière préférée de 5 à 15 % en poids par rapport à la phase (A), et/ou dans laquelle la deuxième phase (B) contenant le deuxième composant principe actif (b) contient du maltitol, en particulier du sirop de maltitol, en particulier en des quantités de 50 à 95 % en poids, en particulier de 60 à 90 % en poids, d'une manière préférée de 70 à 85 % en poids, par rapport à la deuxième phase (B).

12. Composition selon l'une des revendications précédentes, la forme galénique étant une tablette à sucer, en particulier sous forme d'un caramel dur, d'une pastille ou d'une tablette, et/ou la forme galénique étant formée sur la base d'une tablette à sucer, en particulier sous la forme d'une base polyphasique, de préférence diphasique, pour tablette à sucer.

13. Composition selon l'une des revendications précédentes, dans laquelle le rapport en poids entre la première phase solide (A) d'une part et la deuxième phase liquide (B) d'autre part est compris dans la plage de 60-95:40-5, de préférence de 70-90:30-10.

14. Unité de conditionnement, en particulier conditionnement sous blister, contenant la composition selon l'une des revendications précédentes, en particulier sous forme d'une forme posologique unitaire, de préférence sous forme de tablettes à sucer, l'unité de conditionnement contenant en particulier un grand nombre de tablettes à sucer, en particulier pour prélèvement individuel.

15. Utilisation d'une composition telle que définie dans l'une des revendications précédentes pour fabriquer un médicament destiné au traitement topique de maladies inflammatoires de la cavité buccale et du pharynx, de la toux et des catarrhes des voies respiratoires supérieures.
